(19) Europäisches Patentamt
European Patent Office
Office européen des brevets

(11) Numéro de publication: **0 138 720**
**A2**

# (12) DEMANDE DE BREVET EUROPEEN

(21) Numéro de dépôt: **84402097.4**

(22) Date de dépôt: **18.10.84**

(51) Int. Cl.[4]: **C 07 D 295/22**, C 07 D 243/08, C 07 D 307/68, C 07 D 405/06, A 61 K 31/495, A 61 K 31/445, A 61 K 31/535

(30) Priorité: **18.10.83 FR 8316555**

(43) Date de publication de la demande: **24.04.85 Bulletin 85/17**

(84) Etats contractants désignés: **BE CH DE FR GB IT LI LU NL SE**

(71) Demandeur: **DROPIC Société Civile de Gestion de Droit de Propriété Industrielle CHOAY, 10 Avenue Matignon, F-75008 Paris (FR)**

(72) Inventeur: **Roger, Pierre, 6, rue Paul Valéry, F-78423 Montigny Les Bretonneux (FR)**
Inventeur: **Choay, Patrick, 7 Cour Jasmin, F-75016 Paris (FR)**
Inventeur: **Fournier, Jean-Paul, 10, rue Fontenay, F-78000 Versailles (FR)**

(74) Mandataire: **Gutmann, Ernest et al, Cabinet Plasseraud 84, rue d'Amsterdam, F-75009 Paris (FR)**

## (54) Nouveaux benzenesulfonamides n-cyclises, leur procédé de préparation et leur utilisation comme substance active de compositions pharmaceutiques.

(57) L'invention a pour objet de nouveaux benzènesulfonamides N-cyclisés, leur procédé de préparation et leur utilisation comme substance active de compositions pharmaceutiques. Les nouveaux benzènesulfonamides selon l'invention répondant à la formule générale (I) suivante:

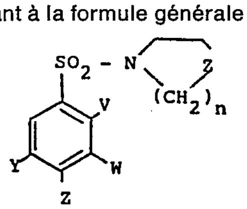

(I)

dans laquelle:

V représente par exemple l'hydrogène,
W représente par exemple $CF_3$,
X représente par exemple l'hydrogène,
Y représente par exemple l'hydrogène,
n vaut 2 ou 3,
Z représente par exemple le groupe $NR_4$, dans lequel $R_4$ représente par exemple l'hydrogène.

Application au traitement de maladies névrotiques avec anxiété.

EP 0 138 720 A2

0138720

# NOUVEAUX BENZENESULFONAMIDES N-CYCLISES, LEUR PROCEDE DE PREPARATION ET LEUR UTILISATION COMME SUBSTANCE ACTIVE DE COMPOSITIONS PHARMACEUTIQUES

L'invention est relative à de nouveaux médicaments qui présentent une activité sur le système nerveux central, notamment anxiolytique, et qui contiennent, à titre de principe actif, des composés présentant une structure de base du type benzènesulfonamide ainsi que leurs sels obtenus avec des acides physiologiquement acceptables.

On appellera ci-après "médicament" toute composition pharmaceutique contenant en association avec un véhicule pharmaceutiquement acceptable l'un au moins des composés chimiques tels que définis ci-après.

L'invention est également relative à un procédé de préparation des susdits composés chimiques ainsi que de leurs sels.

Certains benzènesulfonamides ou leur préparation ont déjà été décrits dans les documents mentionnés ci-après.

Les composés de formule suivante :

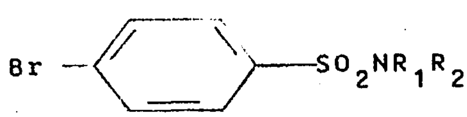

dans laquelle $NR_1R_2$ représente le groupe pipéridino, morpholino, ou méthylpipérazino, sont présentés dans J. Med. Chem., vol. 8, 1965, avec une famille de composés possédant une activité anticonvulsivante. Mais d'après les résultats relatifs à l'activité anticonvulsivante et figurant au tableau I du susdit article, les trois composés particuliers dont les formules respectives viennent d'être explicitées ci-dessus ne présentent pas

l'activité anticonvulsivante de la famille de composés à laquelle ils appartiennent.

Le composé de formule :

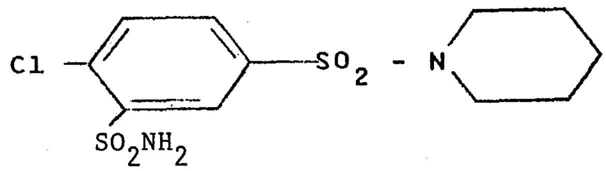

est cité dans l'article Arzneimittelforschung, Heft 6, juin 1967, page 654, comme antidiurétique.

Les composés de formules suivantes :

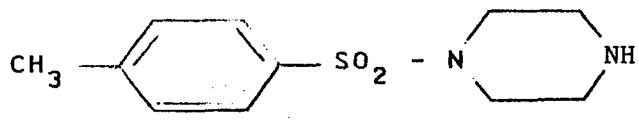

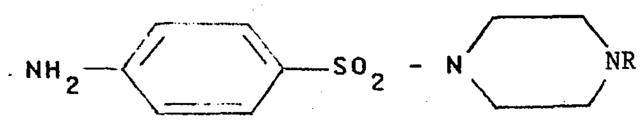

R représentant $CH_3$, $C_2H_5$,

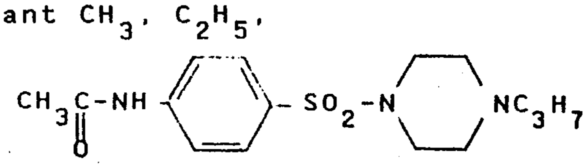

sont connus d'après le brevet US n° 2 415 786, mais aucune indication de leurs propriétés n'est mentionnée.

Le brevet US n° 2 507 408 est relatif aux composés de formule suivante :

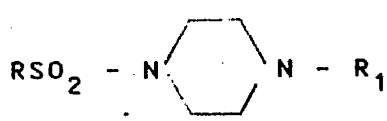

dans laquelle R représente un groupe choisi parmi les radicaux alcoyle contenant de 1 à 4 atomes de carbone, les radicaux phényle qui peuvent être substitués par un membre de la classe constituée par les alcoyle inférieurs, amino, méthoxy, éthoxy, halogène et $R_1$ représente un radical alcoyle ne contenant pas plus de 3 atomes de carbone.

Il est indiqué que l'utilisation de ces composés peut être envisagée pour le traitement des chocs traumatiques et hémorragiques, sans aucune autre précision.

Le composé présenté comme préféré ne possède pas de groupe benzène puisqu'il s'agit de l'éthanesulfonyl-1-éthyl-4-pipérazine.

Le brevet US n° 3 829 487 est relatif aux composés de formule :

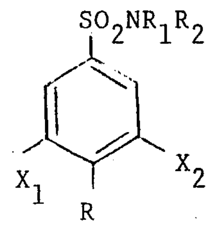

dans laquelle R représente un atome d'hydrogène ou un groupe amino, $X_1$ et $X_2$ représentent chacun un atome d'halogène, un groupe trifluorométhyle ou nitro, $R_1$ et $R_2$ représentent un atome d'hydrogène, un radical alcoxy inférieur, ou un radical alcoyle inférieur substitué dans lequel les substituants sont des groupes alcoxy inférieurs, alcoylthio inférieurs, alcoylsulfinyl inférieurs, alcoylsulfonyl inférieurs, cyano, halogène, ou bien $R_1$ et $R_2$ peuvent former avec l'atome d'azote un hétérocycle de 3 à 6 membres, par exemple les hétérocycles saturés contenant un atome d'azote, tels que aziridinyle, azétidinyle, pyrrolidinyle, pipéridinyle.

L'ensemble des composés est présenté comme étant des agents antiparasites et antihelmintiques, avantageusement utilisés à des doses très élevées dans le traitement de la douve chez les ovins et bovins.

Dans les composés préférés, $R_1$ et $R_2$ représentent des radicaux alcoyle inférieurs, mais ne forment pas, en combinaison avec N, un hétérocycle azoté.

Les composés de formule :

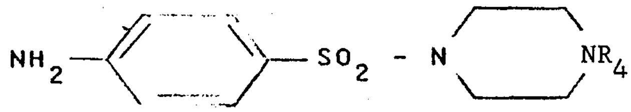

dans laquelle $R_4$ représente un groupe phényle substitué ou non, sont utilisés d'après le brevet US n° 4 159 331 comme produits intermédiaires dans la synthèse des composés (quinolinylaminophényl)sulfonyl-4 pipérazine substitués en 1.

Les composés de formule :

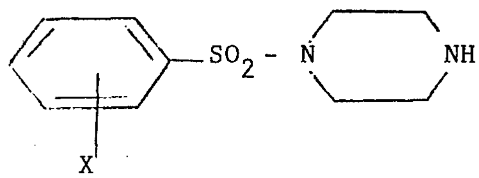

dans laquelle X représente Cl, $CH_3$, $NO_2$, $NH_2$ sont présentés comme ayant une activité analgésique ainsi qu'une activité pharmacologique dans le système cardiovasculaire, sans aucune autre précision.

La préparation du composé de formule :

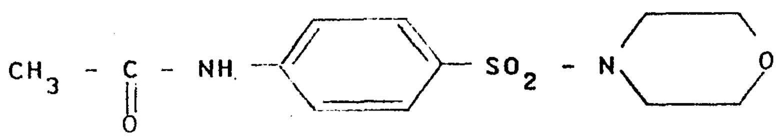

est décrit dans le brevet suisse n° 302 372, mais rien n'est indiqué sur les propriétés de ce composé.

Les Chemical Abstracts, vol. 77, 1972, page 411, 113 957a mentionnent les composés de formule : $2,5\text{-}(CH_3O)_2C_6H_5SO_2R$ dans laquelle R peut représenter un groupe morpholino ou pipéridino.

Mais rien n'est indiqué sur les propriétés de ces composés.

La préparation du composé de formule :

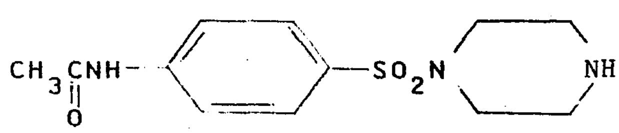

est décrit dans le brevet allemand n° 874 443 ; ce composé est présenté comme ayant une activité bactéricide,

sans aucune autre précision.

Le composé de formule :

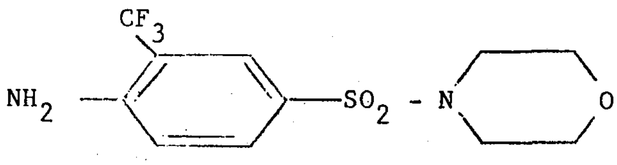

est présenté dans la DOS n° 23 35950 comme intermédiaire de synthèse de produits colorants.

La synthèse du composé 4-aminobenzène-sulfonyl pipéridine est indiquée dans le brevet FR n° 816 988 ; ce composé est présenté comme appartenant à une famille de composés ayant une activité anti-streptococcique et anti-staphylococcique, sans aucune autre précision.

Les médicaments selon l'invention sont caractérisés en ce qu'ils contiennent comme substance active l'un au moins des composés répondant à la formule générale (I) suivante :

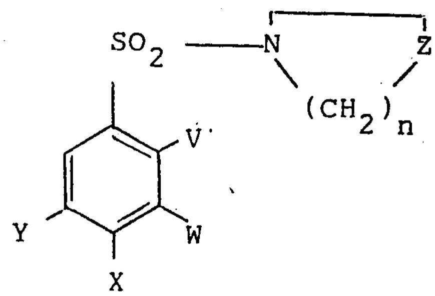

(I)

dans laquelle :

- V représente l'hydrogène, un radical $OR_1$ dans lequel $R_1$ représente un groupe alcoyle de 1 à 4 atomes de carbone ;
- W représente l'hydrogène, le groupe $CF_3$ ;
- X représente l'hydrogène, un halogène, le groupe $NO_2$, $NH_2$, un radical $NH-CO-R_2$ dans lequel $R_2$ représente un radical alcoyle de 1 à 4 atomes de carbone ou aryle, un radical $OR_1$ dans lequel $R_1$ représente un radical alcoyle de 1 à 4 atomes de carbone ;
- Y représente l'hydrogène, un halogène, le groupe $NO_2$,

$NH_2$. $CF_3$, un radical NH-CO-$R_2$ dans lequel $R_2$ représente un radical alcoyle de 1 à 4 atomes de carbone ou aryle, un radical $OR_1$ dans lequel $R_1$ représente un radical alcoyle de 1 à 4 atomes de carbone, un radical $SO_2$-$R_3$ dans lequel $R_3$ représente un radical alcoyle de 1 à 4 atomes de carbone,

- n vaut 2 ou 3 ;
- Z représente un atome d'oxygène, le groupe $CH_2$ ou $NR_4$ dans lequel :

. $R_4$ représente :

- un hydrogène,
- un radical alcoyle inférieur de 1 à 6 atomes de carbone,
- un radical hydroxyalcoyle de 1 à 3 atomes de carbone,
- un radical cycloalcanoyle de 3 à 10 atomes de carbone,
- un radical aryle non substitué ou substitué par un halogène, par le groupe $CF_3$, par un radical $OR_1$ dans lequel $R_1$ représente un radical alcoyle ayant de 1 à 4 atomes de carbone,
- un radical aralcoyle dans lequel la chaîne aliphatique présente de 1 à 4 atomes de carbone et peut comporter des groupes C = O et C-OH et dans lequel le groupe aryle est non substitué ou substitué par un halogène, par le groupe $CF_3$, par un radical $OR_1$, dans lequel $R_1$ est un radical alcoyle ayant de 1 à 4 atomes de carbone,
- un radical aroyle ou ses isostères non substitués ou substitués par un halogène, par le groupe $CF_3$, par un radical $OR_1$ dans lequel $R_1$ est un groupe alcoyle de 1 à 4 atomes de carbone ;

et sous réserve que lorsque n vaut 2 :

- soit Y représente un radical $OR_1$ et l'un au moins des éléments X, Y et W est différent de l'hydrogène ;

- soit :

. – 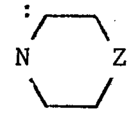 représente un groupe 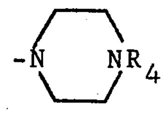

$R_4$ ayant la signification indiquée ci-dessus,

. et Y ou W représente $CF_3$.

En d'autres termes, les composés qui constituent la substance active de ce groupe de médicaments selon l'invention ont une structure, qui, lorsque n vaut 2, comporte :

- soit un groupe pipérazino et un noyau benzénique substitué en méta par un groupe $CF_3$ ;

- soit un groupe pipérazino, morpholino ou pipéridino et un noyau benzénique au moins disubstitué, l'un des deux substituants étant un groupe alcoxy en ortho.

Ce groupe de médicaments sera dans la suite désigné par G1.

L'invention est également relative aux sels d'addition des composés de formule (I) obtenus avec des acides organiques ou minéraux physiologiquement acceptables.

A titre de sels organiques ou minéraux physiologiquement acceptables, on peut citer par exemple le chlorhydrate, le bromhydrate, les sulfates, les phosphates, le méthane sulfonate, l'acétate, le fumarate, le succinate, le lactate, le pyruvate, le citrate, le tartrate, le maléate, le malonate, le benzoate, le salicylate, le dichloro-2,6 benzoate, le triméthoxy benzoate, le diaminobenzène sulfonate, le chromoglycate, le benzène sulfonate, le dipropyl acétate, le glucose-1 phosphate.

Un autre groupe préféré de médicaments selon l'invention est constitué par ceux dont la substance active est un composé de formule (I) :

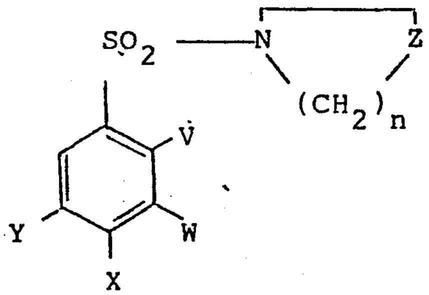

(I)

dans laquelle V,W,X,Y,Z et n ont les significations indiquées ci-dessus et sous réserve que lorsque n vaut 2:

- 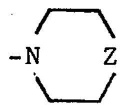 représente un groupe 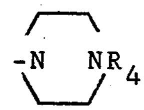

$R_4$ ayant la signification indiquée ci-dessus,

- et :

. soit W ou Y représente un groupe $CF_3$,

. soit V représente un radical $OR_1$ et l'un au moins des éléments X,Y et W est différent de l'hydrogène.

En d'autres termes, les composés qui constituent la substance active de ce groupe de médicaments selon l'invention ont une structure qui, lorsque n vaut 2, comporte :

- soit un groupe pipérazino et un noyau aromatique au moins disubstitué, l'un des substituants étant un radical alcoxy en ortho ;

- soit un groupe pipérazino et un noyau aromatique substitué en méta par $CF_3$.

Ce groupe de composés sera dans la suite désigné par G1bis.

Un groupe préféré de médicaments selon l'invention est constitué par ceux dont la substance active est un composé de formule (I), dans laquelle Z représente O et répond à la formule (II) suivante :

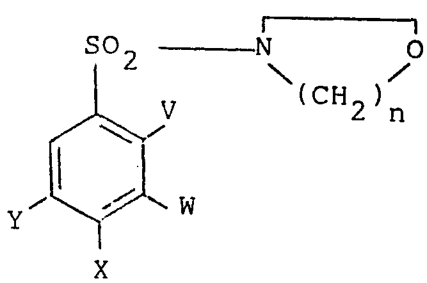

(II)

dans laquelle X, Y, V et W ont les significations indiquées ci-dessus.

Ce groupe sera dans la suite désigné par G2.

Un groupe préféré de médicaments selon l'invention est constitué par ceux dont la substance active est un composé de formule (I), dans laquelle Z représente $CH_2$ et répond à la formule (III) suivante :

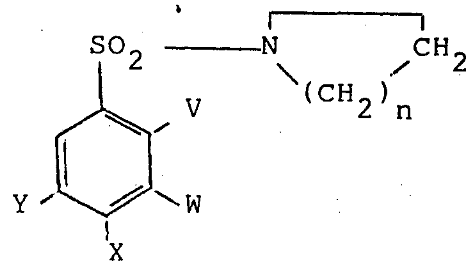

(III)

dans laquelle X, Y, V et W ont les significations indiquées ci-dessus.

Ce groupe sera dans la suite désigné par G3.

Un groupe préféré de médicaments selon l'invention est constitué par ceux dont la substance active est un composés de formule (I), dans laquelle Z représente $NR_4$ et répond à la formule (IV) suivante :

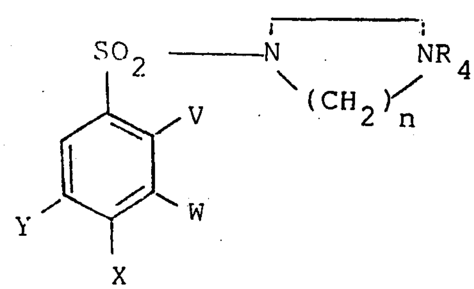

(IV)

dans laquelle X, Y, V, W, $R_4$ et n ont les significations indiquées ci-dessus.

Ce groupe sera dans la suite désigné par G4.

Parmi le groupe G4, une classe préférée de médicaments est constituée par ceux dont la substance active est un composé dans lequel $R_4$ représente un radical cyclopropanoyle, cyclopentanoyle, cyclohexanoyle, adamantoyle, un radical benzoyle ou furoyle.

Ce groupe sera dans la suite désigné par G5.

0138720

Un autre groupe préféré de médicaments selon l'invention est constitue par ceux du groupe G4, dont la substance active est un composé dans lequel $R_4$ représente H et répondant à la formule (VI) suivante :

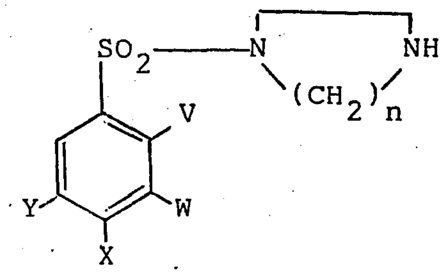

(VI)

dans laquelle X, Y, V, W et n ont les significations indiquées ci-dessus.

Ce groupe sera dans la suite désigné par G6.

Un autre groupe préféré de médicaments selon l'invention est constitué par ceux du groupe G4 dont la substance active est un composé dans lequel Z représente $NR_4$ où $R_4$ représente :

- un radical benzyle non substitué ou substitué par un halogène, par le groupe $CF_3$, par le radical $OR_1$ dans lequel $R_1$ est un alcoyle de 1 à 4 atomes de carbone,

- les groupes $CH_3$, $CH_2CH_2OH$, $CH_2CH_2CH_2OH$, $CH_2CHOHCH_2OH$, $CH_2-C_6H_5$,

$(CH_2)_3-CO$ 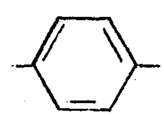 F, 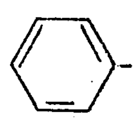 F,

Ce groupe sera dans la suite désigné par G7.

Un groupe préféré de médicaments selon l'invention est constitué par ceux dont la substance active est un composé de formule (I), dans laquelle n vaut 2 et répondant à la formule (VIII) suivante :

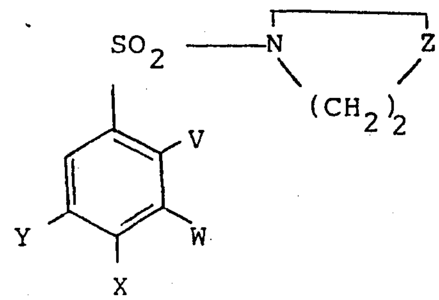

(VIII)

dans laquelle X, Y, V, W et Z ont les significations indiquées ci-dessus.

Ce groupe sera dans la suite désigné par G8.

Un autre groupe préféré de médicaments selon l'invention est constitué par ceux dont la substance active est un composé de formule (I), dans laquelle n vaut 3 et répondant à la formule (IX) suivante :

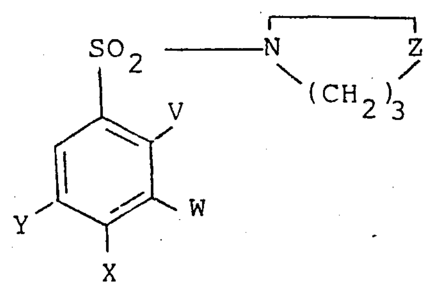

(IX)

dans laquelle X, Y, V et W ont les significations indiquées ci-dessus.

Ce groupe sera dans la suite désigné par G9.

Un autre groupe préféré de médicaments selon l'invention est constitué par ceux dont la substance active est un composé de formule (I), dans laquelle V représente $OCH_3$, W représente H et répondant à la formule (X) suivante :

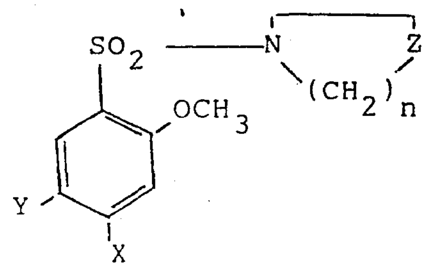

(X)

dans laquelle X représente H, $NO_2$, $OCH_3$, Y représente H, Cl, Br, $OCH_3$, $SO_2CH_3$, $SO_2iC_3H_7$, $CF_3$, Z et n ont les significations indiquées ci-dessus.

Ce groupe sera dans la suite désigné par G10.

Un autre groupe préféré de médicaments selon l'invention est constitué par ceux dont la substance active est un composé de formule (I), dans laquelle V représente H, W représente $CF_3$ et répondant à la formule (XI) suivante :

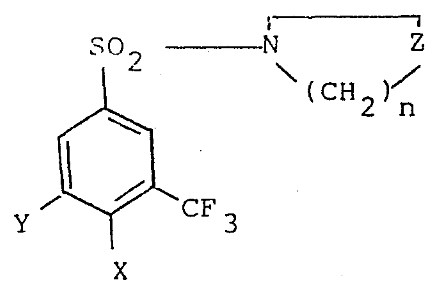

(XI)

dans laquelle X représente H, Cl et Y représente H, $CF_3$, Z et n ont les significations indiquées ci-dessus.

Ce groupe sera dans la suite désigné par G11.

Parmi le groupe G10, un groupe préféré de médicaments selon l'invention est constitué par ceux dont la substance active est un composé de formule (I), dans laquelle V représente $OCH_3$, W représente H, X représente $OCH_3$ et répondant à la formule (XII) suivante :

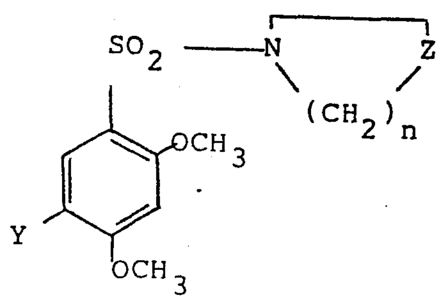

(XII)

dans laquelle Y représente H, $SO_2iC_3H_7$, Br, Cl, $SO_2CH_3$, $OCH_3$, Z et n ont les significations indiquées ci-dessus.

Ce groupe sera dans la suite désigné par G12.

Parmi le groupe G10, un autre groupe préféré de médicaments selon l'invention est constitué par ceux

dont la substance active est un composé de formule (I), dans laquelle V représente $OCH_3$, W représente H, X représente H et répondant à la formule (XIII) suivante :

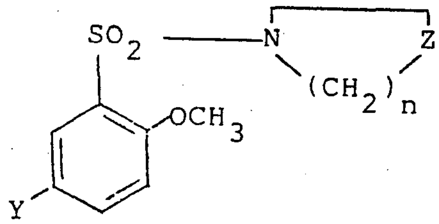

(XIII)

dans laquelle Y représente Br ou Cl, Z et n ont les significations indiquées ci-dessus.

Ce groupe sera dans la suite désigné par G13.

Parmi le groupe G10, un autre groupe préféré de médicaments selon l'invention est constitué par ceux dont la substance active a la formule (I), dans laquelle V représente $OCH_3$, W représente H, X a les significations indiquées dans la définition de G10, mais est différent de l'hydrogène, Y représente l'hydrogène, et répondant à la formule (XIIIa) suivante :

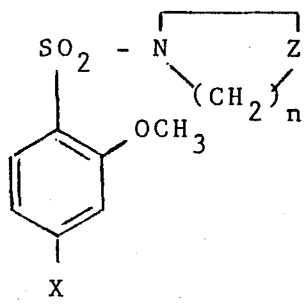

(XIIIa)

dans laquelle Z a la signification indiquée ci-dessus.

Ce groupe sera dans la suite désigné par G13a.

Parmi le groupe G10, un autre groupe préféré de médicaments selon l'invention est constitué par ceux dont la substance active est un composé de formule (I), dans laquelle V représente $OCH_3$, W représente H, X représente $NO_2$ et répondant à la formule (XIV) suivante :

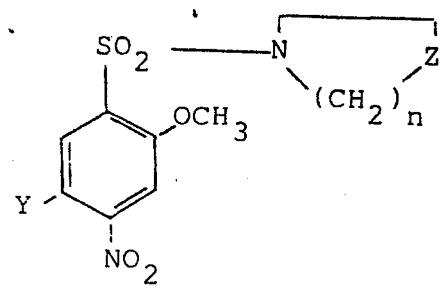

(XIV)

dans laquelle Y représente H ou Cl, Z et n ont les significations indiquées ci-dessus.

Ce groupe sera dans la suite désigné par G14.

Parmi le groupe G11, un autre groupe préféré de médicaments selon l'invention est constitué par ceux dont la substance active est un composé de formule (I), dans laquelle V représente H, W représente $CF_3$, X représente H et répondant à la formule (XV) suivante :

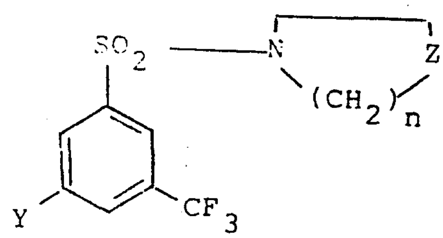

(XV)

dans laquelle Y représente H ou $CF_3$, Z et n ont les significations indiquées ci-dessus.

Ce groupe sera dans la suite désigné par G15.

Parmi le groupe G11, un groupe préféré de médicaments selon l'invention est constitué par ceux dont la substance active est un composé de formule (I) dans laquelle V représente H, W représente $CF_3$, X représente Cl, Y représente H et répond à la formule (XVa) suivante :

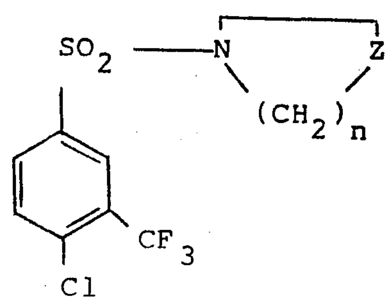

(XVa)

et Z et n ont les significations indiquées ci-dessus.

Parmi le groupe G1, un groupe préféré de médicaments est constitué par ceux de formule (I) dans laquelle V, X et Y ont les significations indiquées dans la définition de G1 et W représente $CF_3$, répondant à la formule générale (XVb) suivante :

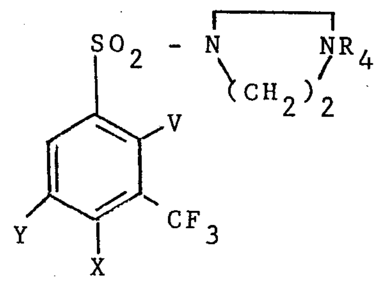

(XVb)

dans laquelle $R_4$ a la signification indiquée ci-dessus.

Ce groupe sera dans la suite désigné par G15b.

Parmi le groupe G1, un groupe préféré de médicaments selon l'invention est constitué par ceux de formule (I), dans laquelle V, X et Y ont les significations indiquées dans la définition de G1, W représente $CF_3$ et répondant à la formule (XVc) suivante :

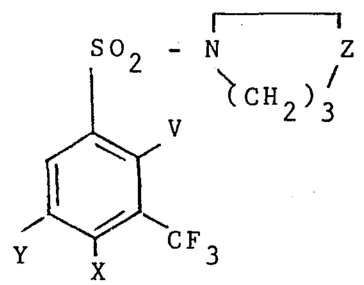

(XVc)

dans laquelle Z a la signification indiquée ci-dessus.

Ce groupe sera dans la suite désigné par G15c.

Un groupe préféré de médicaments selon l'invention est constitué par ceux dont la substance active est un composé répondant à la formule suivante :

Composés n°

1 211

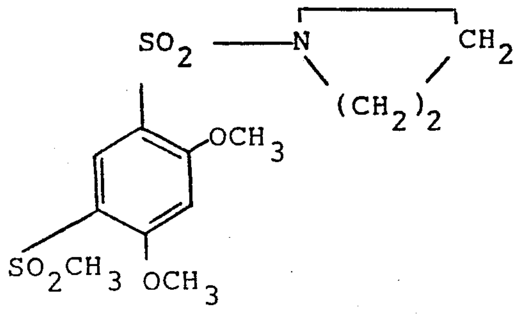

1 224

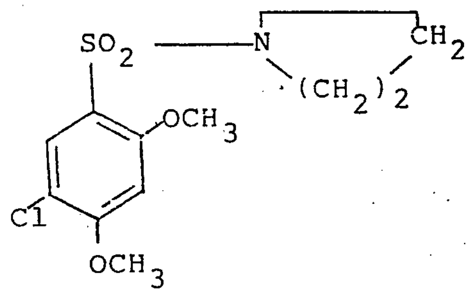

1 242

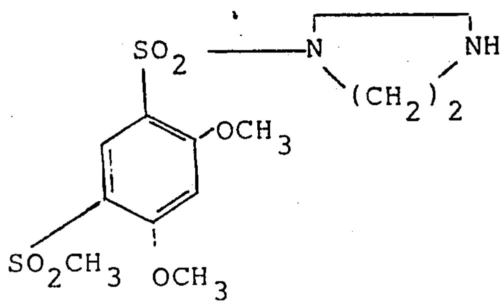

1 247

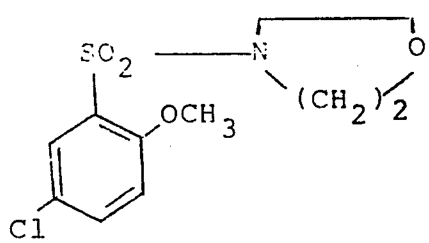

1 265

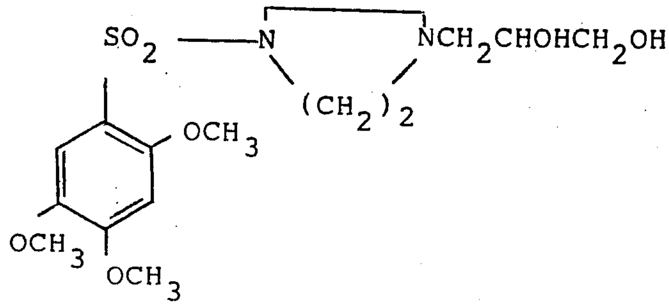

1 270

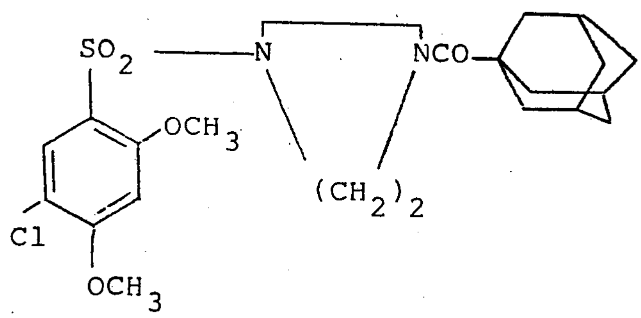

1 301

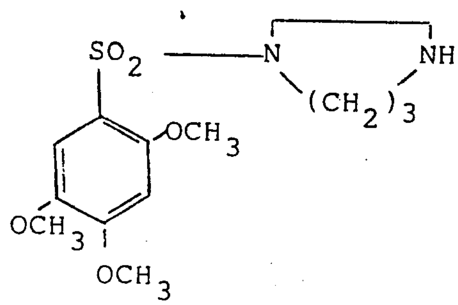

1 304

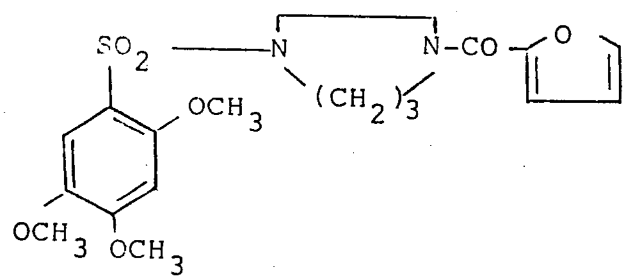

1 306 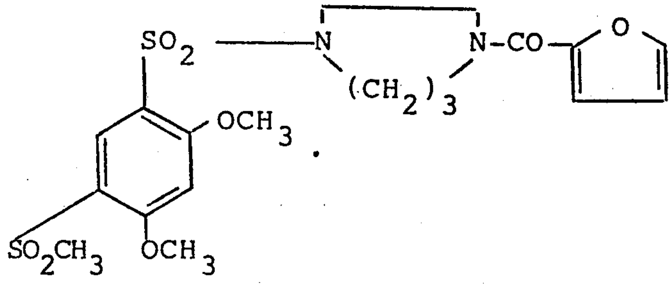 .

Un groupe préféré de médicaments selon l'invention est constitué par ceux dont la substance active est un composé répondant à la formule suivante :

Composés n°

1 334 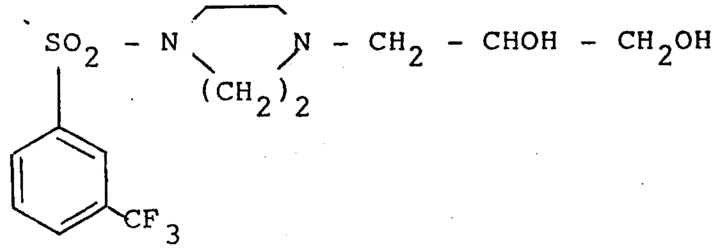

1 400 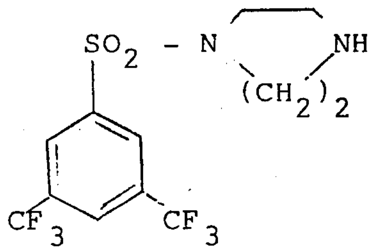

1 399 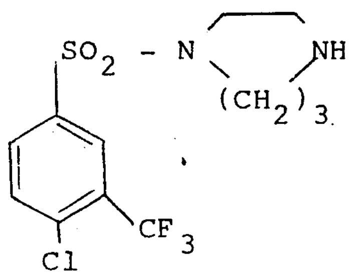

1 389 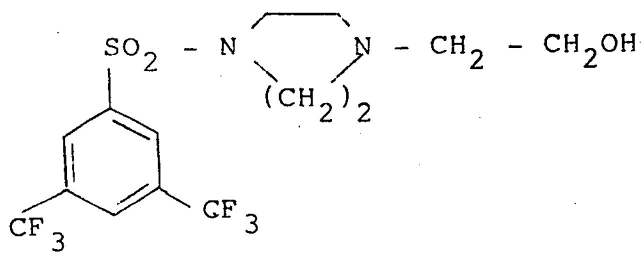

1 397 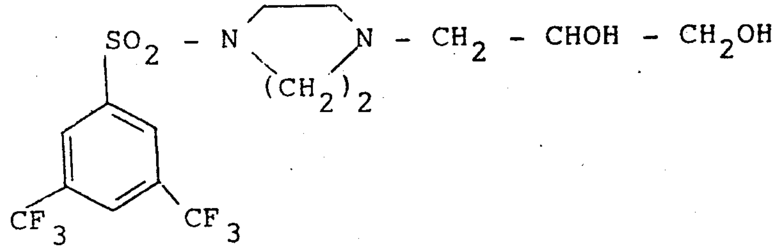

1 390

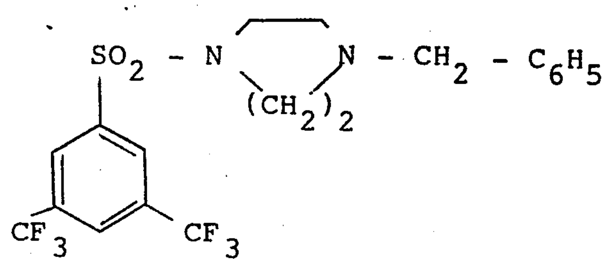

1 336

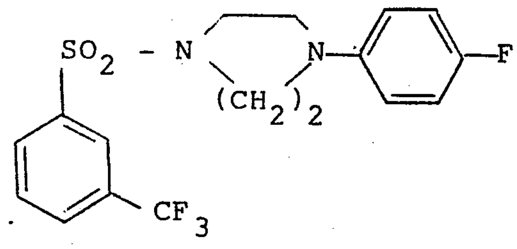

1 392

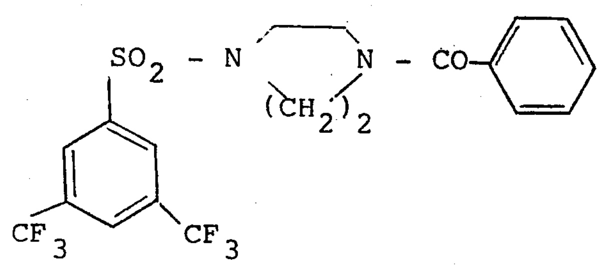

1 393

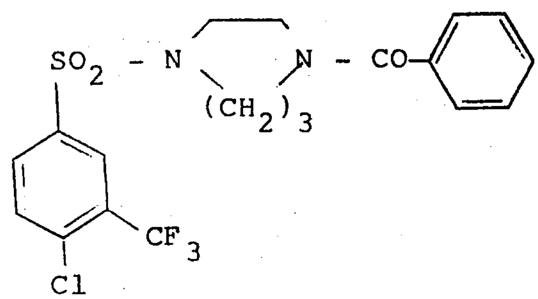

1 402

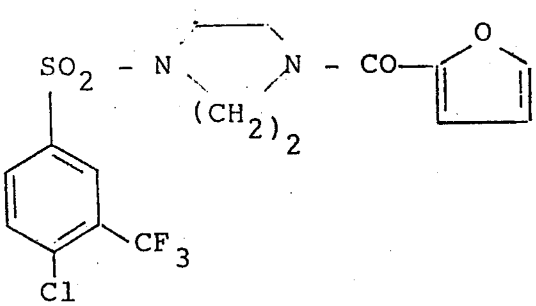

1 396

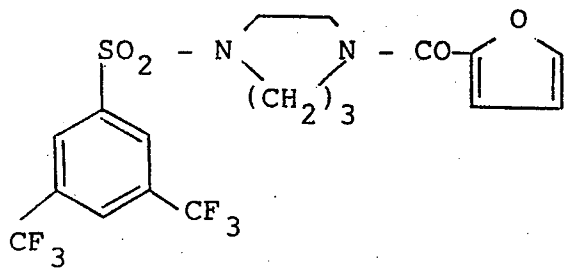

1 404

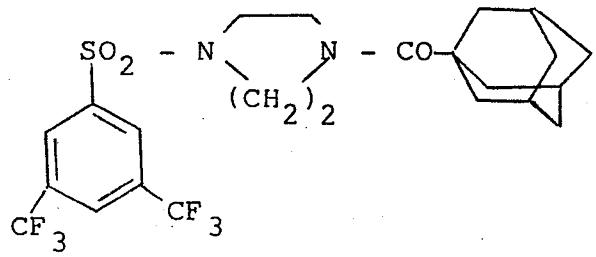

1 407

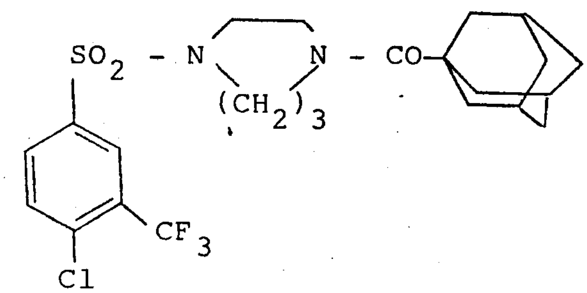

Un groupe préféré de médicaments selon l'invention est constitué par les composés de formules :

Composés n°

1 426

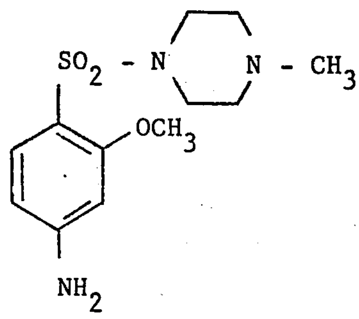

1 406

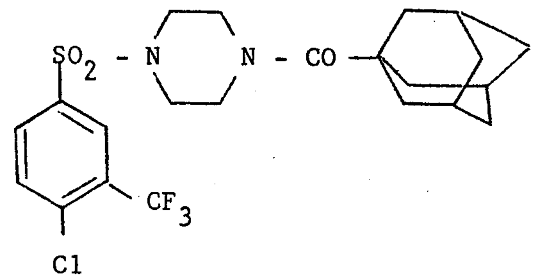

1 505

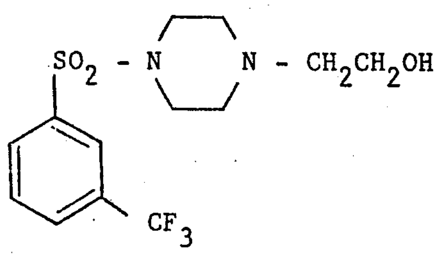

1 260

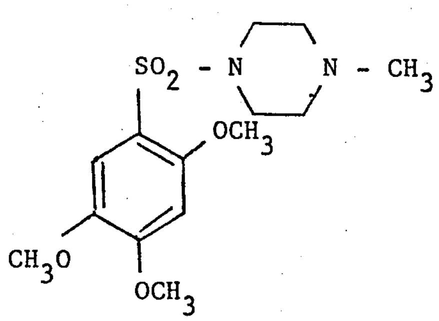

1 299

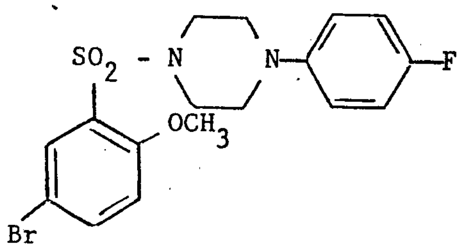

1 403

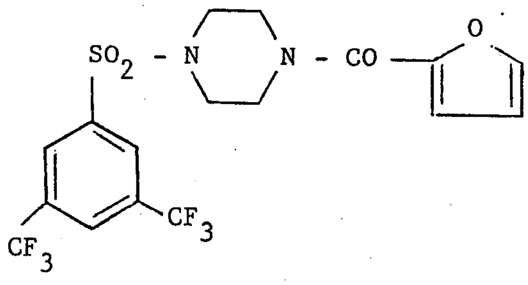

1 408

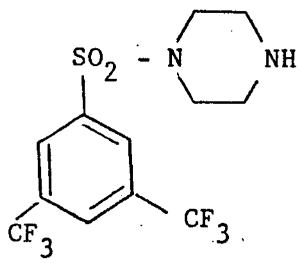

Les composés entrant dans les compositions pharmaceutiques (médicaments) constituent des produits industriels nouveaux, sous réserve que, lorsque n vaut 2 :

- 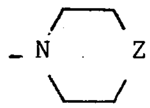 représente un groupe 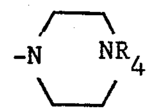

$R_4$ ayant les significations indiquées ci-dessus et

- soit : W ou Y représente $CF_3$

soit : V représente un groupe $OR_1$ dans lequel $R_1$ représente un groupe alcoyle de 1 à 4 atomes de carbone et l'un au moins des éléments W, X ou Y est différent de l'hydrogène.

Font donc partie de l'invention et sont revendiqués les produits nouveaux correspondant aux groupes respectifs de médicaments ci-dessus définis, avec la condition, lorsque n vaut 2, que :

- 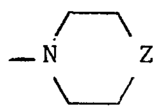 est un groupe pipérazino et

le noyau benzénique est substitué en méta par $CF_3$ ou bien que

- 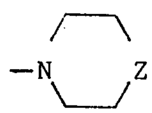 représente un groupe pipérazino

et le noyau benzénique est au moins disubstitué, l'un des substituants étant un groupe alcoxy en position ortho.

Par conséquent, les produits industriels nouveaux de l'invention correspondent aux composés entrant comme substance active dans les compositions pharmaceutiques définies ci-dessus, sous réserve que, lorsque n vaut 2 :

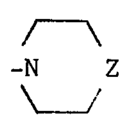 soit différent d'un groupe pipéridino et

morpholino et que le noyau benzénique soit substitué par

un méta $CF_3$ ou au moins disubstitué, l'un des substituants étant un groupe alcoxy.

Les produits industriels nouveaux selon l'invention correspondent aux groupes G1bis, G9, G15b, G15c des médicaments ci-dessus définis.

Les classes de composés préférés selon l'invention sont constituées par les composés appartenant aux classes G2 et G3, sous réserve que n soit égal à 3.

D'autres classes de composés préférés selon l'invention sont constituées par les composés appartenant aux classes G4, G5, G6, G7, sous réserve que lorsque n est égal à 2 :

- soit V représente $OR_1$, $R_1$ ayant les significations indiquées ci-dessus, et l'un au moins des éléments W, X et Y est différent de l'hydrogène ;

- soit W représente $CF_3$.

D'autres classes de composés préférés selon l'invention sont constituées par les composés appartenant à la classe G8, sous réserve que Z représente $NR_4$, $R_4$ ayant les significations indiquées ci-dessus et

- soit V représente $OR_1$, $R_1$ ayant les significations indiquées ci-dessus et l'un au moins des éléments W, X et Y est différent de l'hydrogène ;

- soit W représente $CF_3$.

D'autres classes de composés préférés selon l'invention sont constituées par les composés appartenant à la classe G10, sous réserve que lorsque n est égal à 2, Z représente $NR_4$, $R_4$ ayant les significations indiquées ci-dessus, l'un au moins des éléments X et Y est différent de l'hydrogène.

D'autres classes de composés préférés selon l'invention sont constituées par les composés appartenant aux classes G11, G12, G13, G13a, G14, G15, G15a, sous réserve que lorsque n est égal à 2, Z représente $NR_4$, $R_4$ ayant la signification indiquée ci-dessus.

Les composés particulièrement préférés de l'invention sont ceux de formule :

Composés n°

1 242

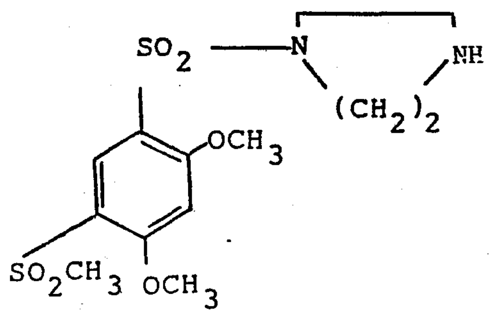

1 265

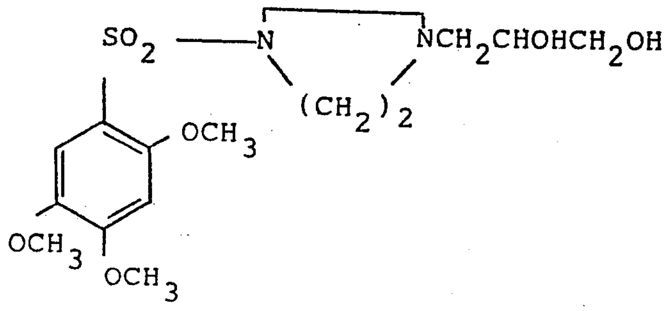

1 270

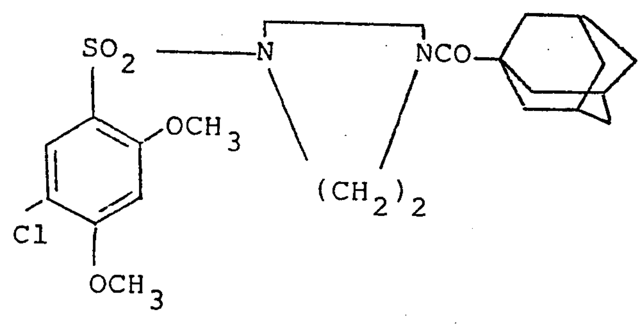

1 301

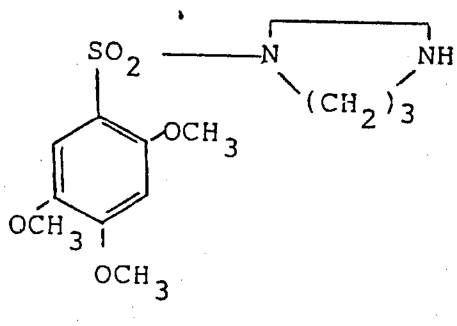

1 304

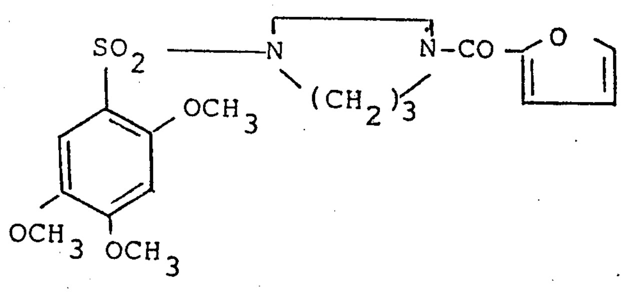

1 306

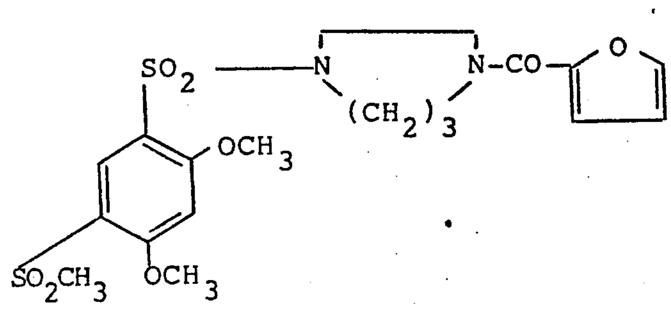

1 334

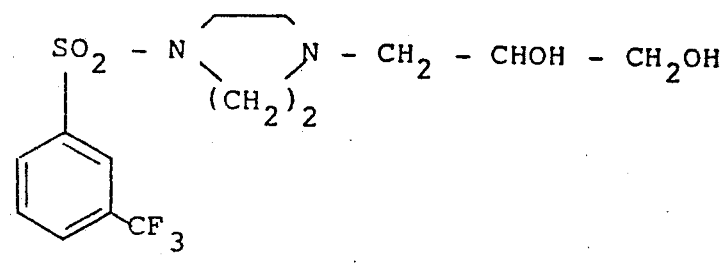

1 400

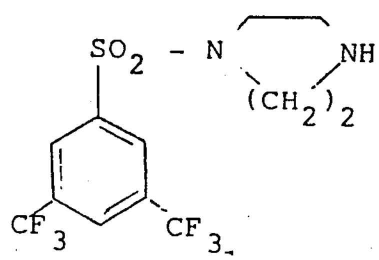

1 399

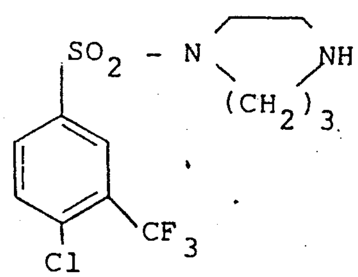

1 389

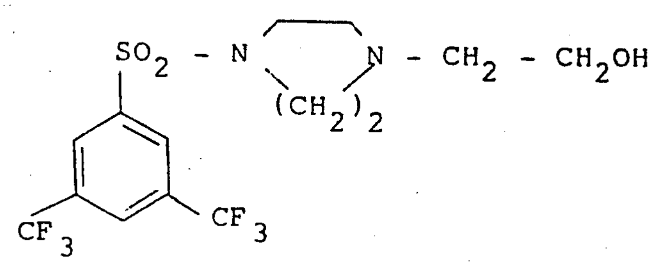

1 397

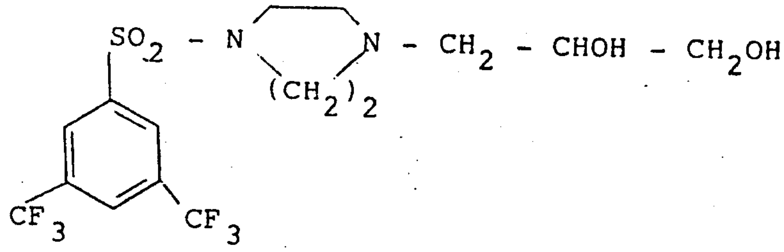

1 390

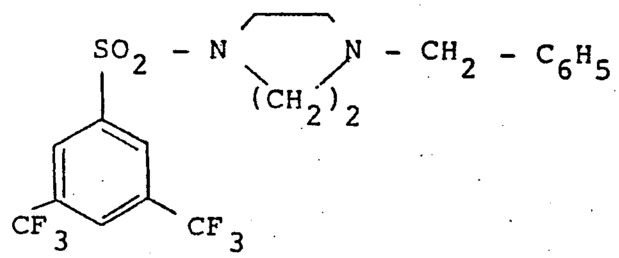

1 336

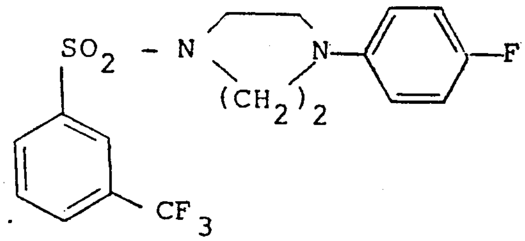

1 392

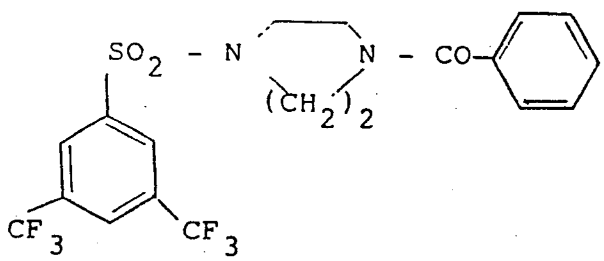

1 393

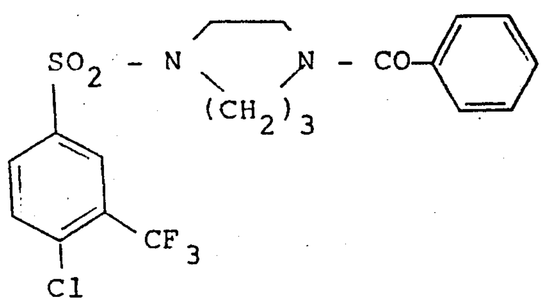

1 402

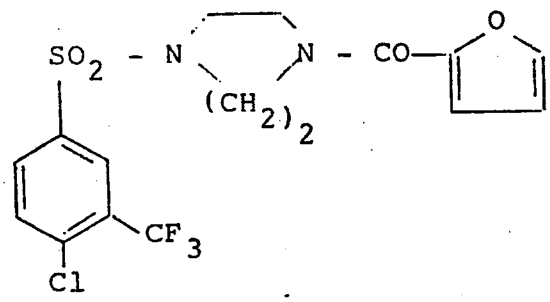

1 396

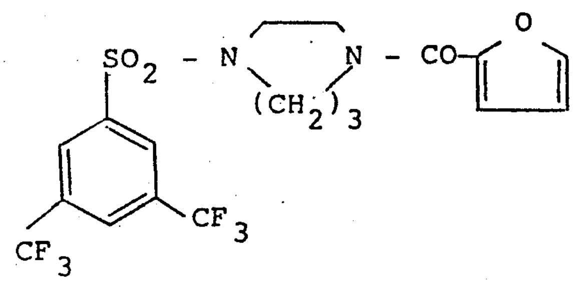

1 404

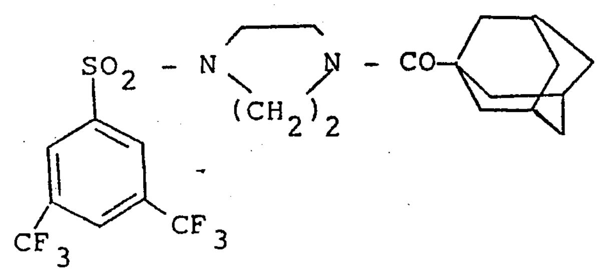

1 407

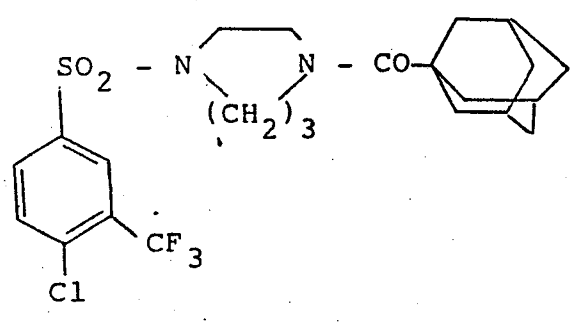

Composés n°

1 426 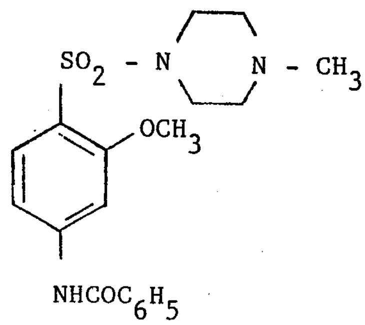

1 406 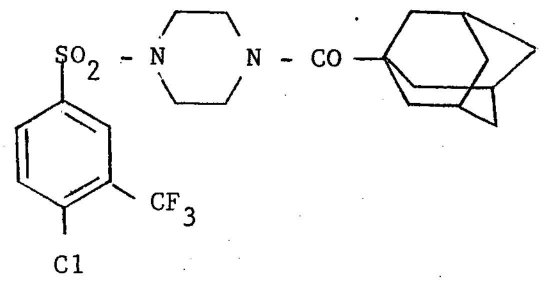

1 505 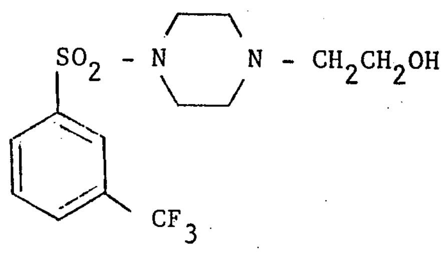

1 260 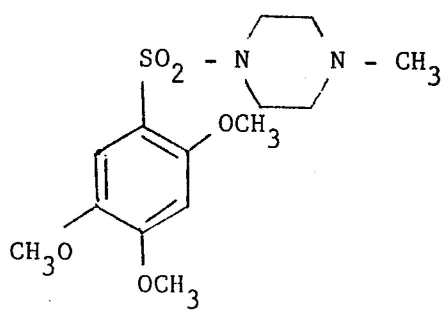

1 299

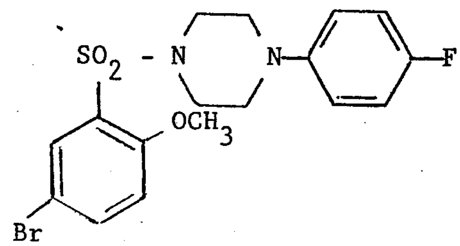

1 403

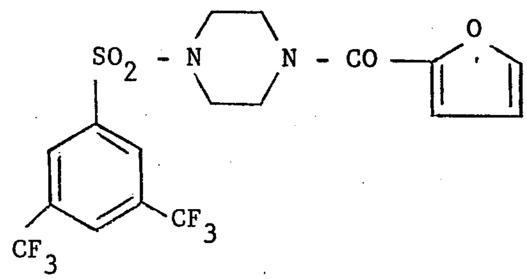

1 408

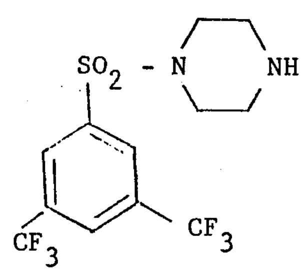

SYNTHESE DES COMPOSES ENTRANT DANS LA COMPOSITION DES MEDICAMENTS SELON L'INVENTION AINSI QUE DES COMPOSES NOUVEAUX SELON L'INVENTION

Dans ce qui suit, on décrit la synthèse des composés entrant dans la composition des médicaments selon l'invention, ainsi que celle des composés nouveaux selon l'invention.

Les composés de formule (I) peuvent être obtenus par réaction du sulfohalogénure de formule (XVI) :

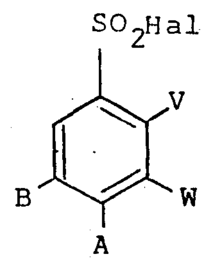

(XVI)

dans lequel V, W ont les significations indiquées ci-dessus, A et B ont les significations de X et Y, à l'exception de $NH_2$, $NH\underset{\underset{O}{\|}}{C}R_2$

et Hal représente un atome d'halogène, notamment brome ou chlore, sur le composé de formule (XVII) :

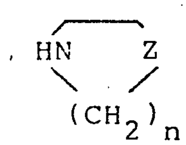

(XVII)

dans laquelle Z et n ont les significations indiquées ci-dessus.

Pour effectuer cette réaction, on utilise de préférence le composé de formule (XVII) soit en excès par rapport au composé de formule (XVI), soit en ajoutant une amine tertiaire, telle que la triéthylamine, comme accepteur de l'hydracide, afin d'obtenir le composé de formule (I) sous forme non salifiée.

L'obtention des composés de formule (I) dans laquelle X ou Y représente $NH_2$ est réalisée en réduisant le composé de formule (Ia) :

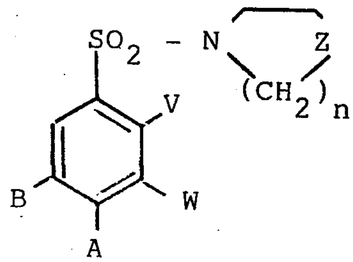

(Ia)

dans laquelle A ou B représente $NO_2$ par hydrogénation catalytique ou par réduction chimique.

L'obtention des composés de formule (I), dans laquelle X ou Y représente $NHCOR_2$, peut être réalisée dans une première étape en réduisant le composé de formule (Ia), comme indiqué ci-dessus, pour transformer le groupe $NO_2$ correspondant à A ou B en $NH_2$, puis dans une seconde étape en faisant réagir sur le composé obtenu à l'issue de la première étape, $R_2COCl$ ou $(R_2CO)_2O$, $R_2$ représentant un groupe alcoyle de 1 à 4 atomes de carbone, pour transformer le groupe $NH_2$ en $NHCOR_2$.

Pour former les composés selon l'invention, on peut avoir recours aux sulfohalogénures, notamment aux sulfochlorures de formule (XVIII) :

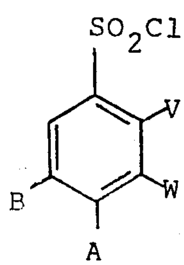

, (XVIII)

dans laquelle V, W ont les significations indiquées ci-dessus, A et B ont respectivement les significations de X et Y à l'exception de $NH_2$ ou $NH\underset{\underset{O}{\|}}{C}R_2$.

La préparation des sulfochlorures est soit décrite dans la littérature, notamment dans les brevets FR n° 2 313 918 et 2 338 929, soit pour les sulfochlorures de formule (XIX) :

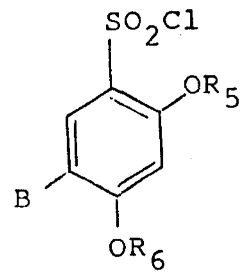

(XIX)

dans laquelle $R_5$ et $R_6$ représentent, indépendamment l'un de l'autre, un radical alcoyle de 1 à 4 atomes de carbone, décrite dans les demandes de brevet France n° 2 504 528 et 2 504 527, déposées le 23 avril 1981.

Pour préparer les composés de formule (VI) :

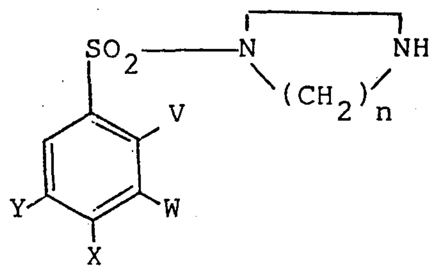

(VI)

on peut également faire réagir un sulfochlorure de formule (XVIII) :

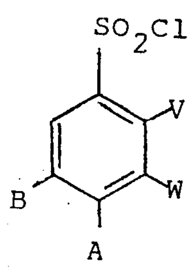

(XVIII)

sur le composé de formule (XX) :

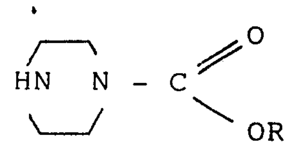

(XX)

dans lequel R représente un alcoyle de 1 à 4 atomes de carbone, de préférence le radical éthyle, ce qui conduit au composé de formule (XXI) :

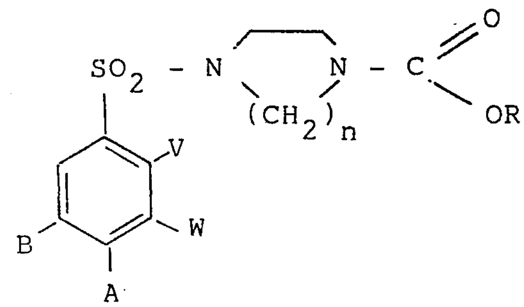

(XXI)

suivi d'une hydrolyse alcaline selon des méthodes classiques, telles que par une base forte telle que KOH, et éventuellement transformer A ou B en $NH_2$, comme indiqué ci-dessus, pour obtenir le composé de formule (VI).

Cette synthèse sera dans la suite désignée par synthèse 1.

Pour préparer les composés de formule (VI), on peut également faire réagir le sulfochlorure de formule (XVIII) :

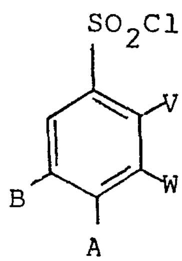

(XVIII)

sur le composé de formule (XXII) :

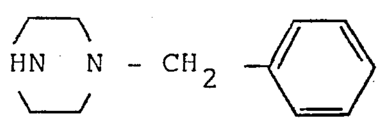

(XXII)

pour obtenir le composé de formule (XXIII) :

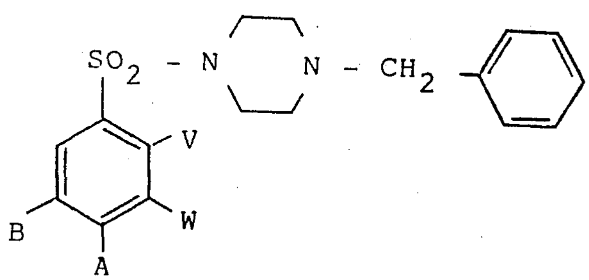

(XXIII)

puis éliminer le radical benzyle selon des méthodes classiques, telles que par hydrogénolyse sur palladium et sur charbon.

On peut utiliser de préférence ce procédé lorsque le noyau aromatique ne comporte pas d'halogène.

Cette synthèse sera dans la suite désignée par synthèse 2.

Pour obtenir le composé de formule (IV) :

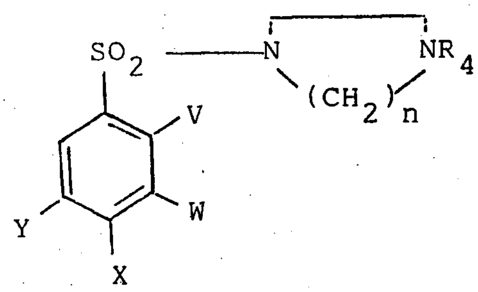

(IV)

dans laquelle $R_4$ représente

- un radical hydroxyalcoyle de 2 à 3 atomes de carbone ;

- un radical aralcoyle dans lequel la chaine aliphatique présente de 1 à 4 atomes de carbone et peut comporter des groupes C = O et $\underset{OH}{\underset{|}{C}}$ et dans lequel le groupe aryle peut être non substitué ou substitué par un halogène, par le groupe $CF_3$, par un radical $OR_1$, dans lequel $R_1$ est un radical alcoyle ayant de 1 à 4 atomes de carbone ;

- un radical aroyle ou ses isostères non substitués ou substitués par un halogène, par le groupe $CF_3$, par un radical $OR_1$ dans lequel $OR_1$ est un groupe alcoyle de 1 à 4 atomes de carbone ;

on peut fabriquer dans une première étape le composé de formule (XXIV) :

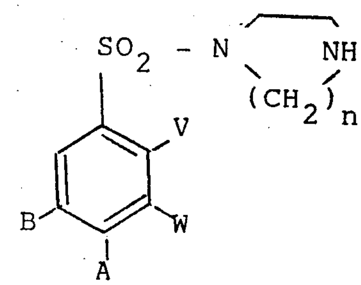

(XXIV)

puis dans une deuxième étape transformer ce composé de formule (XXIV) en composé de formule (IV) respectivement par addition d'un époxy correspondant à l'hydroxyde, d'un chlorure d'aralcoyle correspondant à l'aralcoyle et d'un chlorure d'aroyle correspondant à l'aroyle.

Les trois schémas réactionnels ci-dessous sont donnés à titre d'exemple pour représenter la transformation d'un composé de formule (XXIV) en trois composés de formule (IV), dans lesquels $R_4$ représente respectivement un aralcoyle, un aroyle et un hydroxyalcoyle.

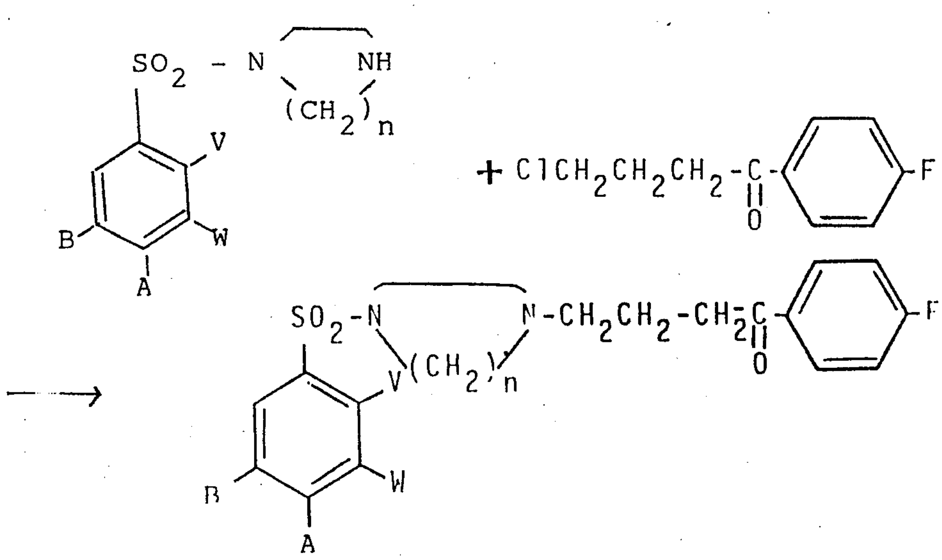

Cette réaction de condensation a lieu pendant 48 heures en présence d'un carbonate alcalin, de préférence de potassium, et en présence d'un solvant tel que la méthylisobutylcétone ou de tout autre solvant dans lequel les produits utilisés sont solubles et dont la température de reflux est voisine de celle de la méthylisobutylcétone.

Cette réaction est éventuellement suivie d'une transformation de A ou B en $NH_2$.

Cette synthèse sera dans la suite désignée par synthèse 3.

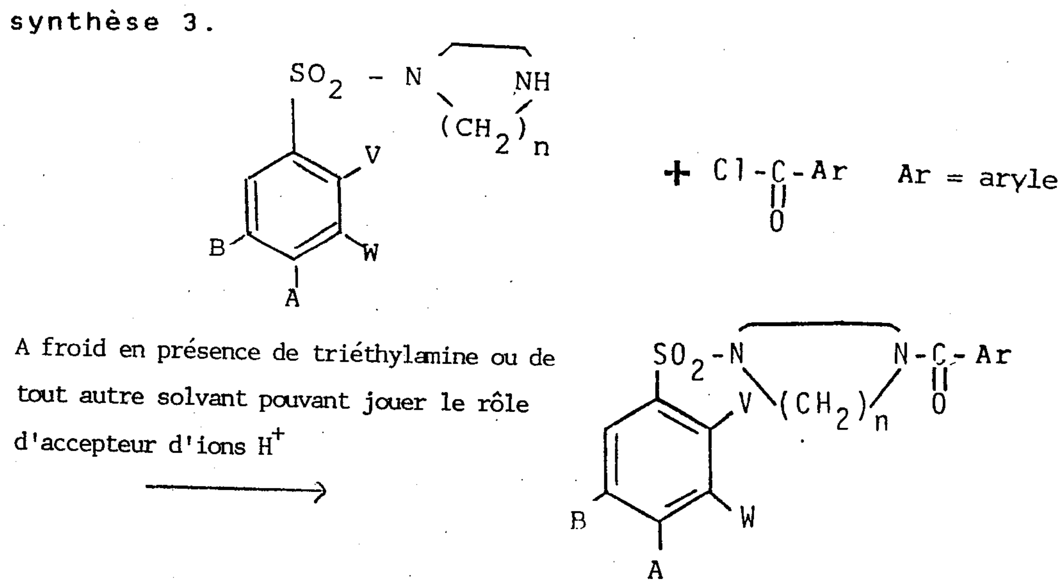


Cette réaction est éventuellement suivie d'une transformation de A ou B en $NH_2$.

Cette synthèse sera dans la suite désignée par synthèse 4.

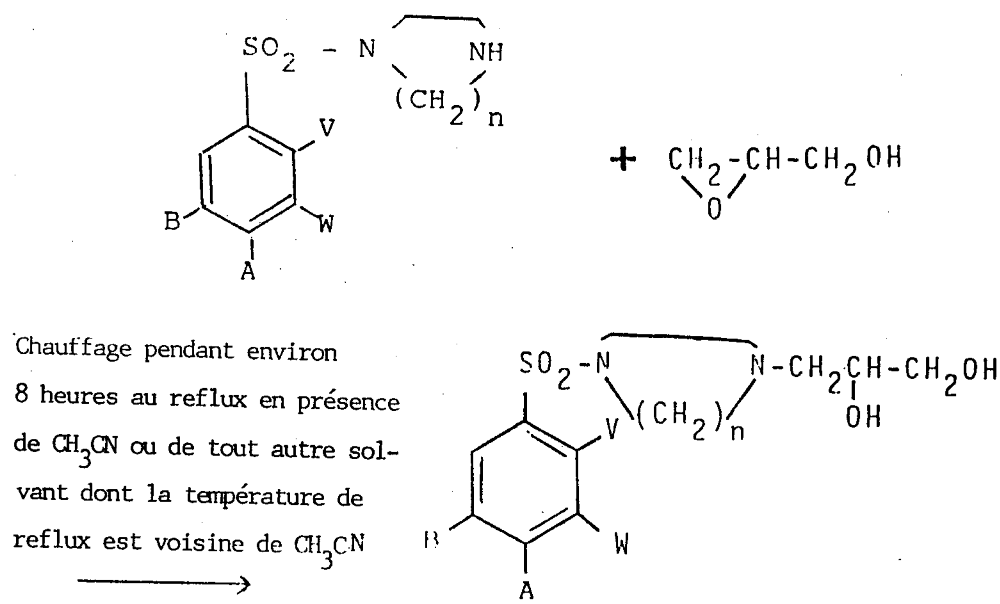


Cette réaction est éventuellement suivie d'une transformation de A ou B en $NH_2$.

Cette synthèse sera dans la suite désignée par synthèse 5.

Selon un mode de réalisation préféré du procédé de préparation des composés selon l'invention, les composés de formule :

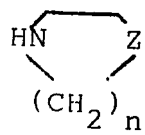

sont de préférence choisis parmi :

- la morpholine,
- la pipéridine,
- la pipérazine,
- l'homopipérazine,
- la N(hydroxy-2)éthyl pipérazine,
- la N-benzylpipérazine,
- la (fluoro-4 phényl)-1 pipérazine,
- la (dihydroxy-2,3 propyl)-1 pipérazine,
- la N-méthyl pipérazine,
- la N-carbéthoxypipérazine,
- l'(hydroxy-3-propyl)-1 pipérazine.

Selon un mode de réalisation préféré du procédé de préparation des composés selon l'invention, les composés de formule :

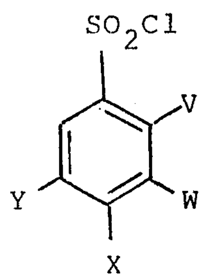

sont de préférence choisis parmi :

- les sulfochlorures figurant dans les brevets FR n° 2 313 918, 2 338 929, 2 504 528, 2 504 527 :
- chlorure de (ditrifluorométhyl-3,5) benzènesulfonyle (voir exemple 1) ;
- chlorure de bromo-5 diméthoxy-2,4 benzènesulfonyle ;

- chlorure de chloro-5 méthoxy-2 benzènesulfonyle ;

- chlorure de chloro-5 diméthoxy-2,4 benzènesulfonyle ;

- chlorure de diméthoxy-2,4 isopropylsulfonyl-5 benzènesulfonyle ;

- chlorure de diméthoxy-2,4 méthylsulfonyl-5 benzènesulfonyle.

Dans le cas des synthèses 3, 4 et 5, les composés que l'on fait réagir sur les composés de formule :

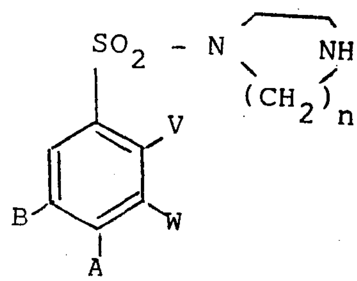

sont de préférence choisis parmi la chloro p. fluorobutyrophénone, le chlorure de benzoyle, le chlorure d'adamantoyle, l'époxy-2,3 propanol-1.

Pour obtenir les sels physiologiquement acceptables des composés entrant dans la composition des médicaments selon l'invention, on salifie selon des méthodes classiques.

Les exemples suivants relatifs à la préparation d'un certain nombre de composés entrant dans la composition des médicaments selon l'invention servent d'illustration, mais ne sont pas limitatifs.

EXEMPLE 1

Cet exemple est relatif à la préparation du chlorure de (ditrifluorométhyl-3,5) benzènesulfonyle.

46 g (0,2 mole) de ditrifluorométhyl-3,5 aniline sont ajoutés à un mélange de 150 ml d'une solution d'acide chlorhydrique (d : 1,18) et 50 ml d'acide acétique pur par petites portions.

Le mélange est mis sous agitation puis refroidi

vers -5°C. Une solution de nitrite de sodium (14,5 g dans 50 ml d'eau) est ajoutée goutte à goutte en maintenant la température en-dessous de 0°C. L'addition terminée, on laisse en contact sous agitation à -5°C, pendant une heure puis on filtre la solution.

Le filtrat est ajouté par portions à une solution saturée d'anhydride sulfureux dans l'acide acétique (500 ml) contenant 11 g de chlorure cuivrique.

Le mélange est laissé sous agitation vive pendant 2H30, puis de la glace pilée est ajoutée jusqu'à précipitation du sulfochlorure.

Le précipité essoré est lavé à l'eau puis séché sous vide.

On a obtenu 51,4 g de produit.

Rendement : 82 % - F° : 38°C

EXEMPLE 2

[ (Bromo-5 diméthoxy-2,4) benzènesulfonyl ]-1 morpholine (composé n° 1 216)

A 9,47 g (0,03 mole) de chlorure de bromo-5 diméthoxy-2,4 benzènesulfonyle dans 50 ml de chlorure de méthylène anhydre sont ajoutés, à 0°C et sous agitation, 5,22 g (0,06 mole) de morpholine. Après quatre heures d'agitation à température ambiante, le milieu réactionnel est évaporé à sec sous vide. Le résidu est repris par 20 ml d'eau. Les cristaux sont essorés, lavés à l'eau jusqu'à absence d'ion $Cl^-$, séchés, puis recristallisés dans l'acide acétique.

On a obtenu 8,36 g de produit.

Rendement : 76 % - F° : 186°C

Analyse : $C_{12}H_{16}BrNO_5S$ : 366,23 - Calculé C : 39,3 ; H : 4,4 ; N : 3,8 - Trouvé C : 39,2 ; H : 4,3 ; N : 4,0.

EXEMPLE 3

[ (Bromo-5 diméthoxy-2,4) benzènesulfonyl ]-1-pipéridine (composé n° 1 218)

A 9,47 g (0,03 mole) de chlorure de bromo-5 diméthoxy-2,4 benzènesulfonyle dans 50 ml de chlorure de

méthylène anhydre sont ajoutés, à 0°C et sous agitation, 5,10 g (0,06 mole) de pipéridine. Le traitement est le même qu'à l'exemple 2.

On a obtenu 8,39 g de produit.

Rendement : 77 % - F° : 149°C

Analyse : $C_{13}H_{18}BrNO_4S$ : 364,26 - Calculé C : 42,8 ; H : 4,9 ; N : 3,8 - Trouvé C : 42,6 ; H : 4,8 ; N : 3,8.

EXEMPLE 4

[ (Chloro-5 méthoxy-2) benzènesulfonyl ]-1 pipérazine (composé n° 1 240)

A 43 g (0,5 mole) de pipérazine dans 250 ml de chlorure de méthylène, on ajoute en refroidissant à 0°C, et en 4 heures, une solution de 24,1 g (0,1 mole) de chlorure de chloro-5 méthoxy-2 benzènesulfonyle dans 150 ml de chlorure de méthylène anhydre. L'addition terminée, le mélange réactionnel est ramené à température ambiante et l'agitation maintenue pendant 6 heures. Après évaporation à sec sous vide, le résidu est repris dans 200 ml d'acide chlorhydrique N. L'insoluble est éliminé. Par addition de soude N, on amène le pH à 9. Le précipité est essoré, lavé à l'eau, puis séché. Les cristaux sont recristallisés dans le benzène.

On a obtenu 19,42 g de produit.

Rendement : 67 % - F° : 145°C

Analyse : $C_{11}H_{15}ClN_2O_3S$ : 290,76 - Calculé C : 45,4 ; H : 5,2 ; N : 9,6 - Trouvé C : 45,5 ; H : 5,1 ; N : 9,5.

EXEMPLE 5

[ (Hydroxy-2) éthyl ]-4 [ (chloro-5 diméthoxy-2,4) benzènesulfonyl ]-1 pipérazine (composé n° 1 296)

A 3,9 g (0,03 mole) de N(hydroxy-2) éthyl pipérazine en solution dans 30 ml de chlorure de méthylène anhydre, sont ajoutés, à 0°C et sous agitation, 10 ml de triéthylamine redistillée, puis goutte à goutte, 8,1 g

(0,03 mole) de chlorure de chloro-5 diméthoxy-2,4 benzènesulfonyle en solution dans 60 ml de chlorure de méthylène anhydre. L'addition terminée, le mélange réactionnel est ramené à température ambiante et laissé sous agitation pendant 12 heures. Après évaporation à sec sous vide, le résidu est repris dans l'eau. Les cristaux sont essorés, lavés à l'eau jusqu'à absence d'ion $Cl^-$ puis séchés sous vide. Ils sont recristallisés dans le benzène.

On a obtenu 8,12 g de produit.

Rendement : 74 % - F° : 118°C

Analyse $C_{14}H_{21}ClN_2O_5S$ : 364,86 - Calculé C : 46,1 ; H : 5,8 ; N : 7,7 - Trouvé C : 46,0 ; H : 5,7 ; N : 7,6.

EXEMPLE 6

Benzyl-4 [ (diméthoxy-2,4 isopropylsulfonyl-5) benzènesulfonyl ]-1 pipérazine (composé n° 1 214)

A 3,42 g (0,01 mole) de chlorure de diméthoxy-2,4 isopropylsulfonyl-5 benzènesulfonyle dans 30 ml de chlorure de méthylène anhydre, sont ajoutés 3,52 g (0,02 mole) de N-benzylpipérazine dans 20 ml de chlorure de méthylène anhydre, goutte à goutte, à 0°C et sous agitation. L'addition terminée, le milieu réactionnel est ramené à température ordinaire et agité pendant 12 heures.

Après évaporation à sec sous vide, le résidu est repris par 30 ml d'eau. Les cristaux sont essorés, lavés à l'eau jusqu'à absence d'ion $Cl^-$, séchés puis recristallisés dans le méthanol.

On a obtenu 3,57 g de produit.

Rendement : 74 % - F° : 180°C

Analyse $C_{22}H_{30}N_2O_6S_2$ : 482,60 - Calculé C : 54,7 ; H : 6,3 ; N : 5,8 - Trouvé C - 54,5 ; H : 6,2 ; N : 5,3.

EXEMPLE 7

[ (Fluoro-4) phényl ]-4 [ (diméthoxy-2,4 méthylsulfonyl-5) benzènesulfonyl ]-1 pipérazine (composé n° 1 298)

A 3,15 g (0,01 mole) de chlorure de diméthoxy-2,4 méthylsulfonyl-5 benzènesulfonyle dans 30 ml de chlorure de méthylène anhydre sont ajoutés 5 ml de triéthylamine redistillée puis, goutte à goutte, à 0°C et sous agitation, 1,8 g (0,01 mole) de (fluoro-4 phényl)-1 pipérazine dans 10 ml de chlorure de méthylène anhydre. L'addition terminée, le milieu réactionnel est ramené à température ambiante et laissé sous agitation pendant 12 heures.

Après évaporation sous vide, le résidu est repris dans 20 ml d'eau. Les cristaux essorés, lavés à l'eau jusqu'à absence d'ion $Cl^-$, séchés, sont ensuite recristallisés dans l'acétonitrile.

On a obtenu 3,24 g de produit.

Rendement : 71 % - F° : 255°C

Analyse $C_{19}H_{23}FN_2O_6S_2$ : 458,53 - Calculé C : 49,8 ; H : 5,1 ; N : 6,1 - Trouvé C : 49,7 ; H : 5,1 ; N : 6,1.

Les produits suivants ont été préparés selon l'exemple 2 :

Composés n° 1 202, 1 209, 1 215, 1 216, 1 223, 1 246, 1 247.

Les produits suivants ont été préparés selon l'exemple 3 :

Composés n° 1 208, 1 211, 1 218, 1 224, 1 248, 1 249.

Les produits suivants ont été préparés selon l'exemple 4 :

- avec la pipérazine :

Composés n° 1 204, 1 220, 1 240, 1 241, 1 242, 1 243, 1 400, 1 401, 1 504 ;

- avec l'homopipérazine :

Composés n° 1 301, 1 302, 1 303, 1 399, 1 408, 1 521.

Les produits suivants ont été préparés selon l'exemple 5 :

- avec l'[(hydroxy-2) éthyl]-1 pipérazine :

Composés n° 1 263, 1 264, 1 290, 1 294, 1 296, 1 389, 1 398, 1 505 ;

- avec la (dihydroxy-2,3 propyl)-1 pipérazine : Composés n° 1 265, 1 291, 1 293, 1 295, 1 334, 1 387, 1 397 ;

- avec l'(hydroxy-3-propyl)-1-pipérazine : composé n° 1 500.

Les produits suivants ont été préparés selon l'exemple 6 :

- avec la N-benzylpipérazine : Composés n° 1 206, 1 213, 1 214, 1 219, 1 222, 1 244, 1 245, 1 335, 1 390, 1 391 ;

- avec la N-méthylpipérazine : Composés n° 1 260, 1 261, 1 292, 1 424.

Les produits suivants ont été préparés selon l'exemple 7 : Composés n° 1 297, 1 298, 1 299, 1 300, 1 336, 1 353, 1 388.

EXEMPLE 8

Carbéthoxy-4 [ (chloro-5 diméthoxy-2,4) benzènesulfonyl ]-1 pipérazine

A 27,1 g (0,1 mole) de chlorure de chloro-5 diméthoxy-2,4 benzènesulfonyle en solution dans 250 ml de chlorure de méthylène anhydre sont ajoutés, à 0°C et sous agitation, 50 ml de triéthylamine redistillée puis goutte à goutte, une solution de 15,8 g (0,1 mole) de N-carbéthoxy pipérazine dans 100 ml de chlorure de méthylène anhydre. L'addition terminée, le milieu réactionnel est ramené à température ambiante et laissé sous agitation pendant 4 heures. Après évaporation à sec sous vide, le résidu est repris dans 100 ml d'eau. Les cristaux sont essorés, lavés à l'eau, séchés, puis recristallisés dans le méthanol.

On a obtenu 33 g de produit.

Rendement : 84 % - F° : 153°C

Analyse $C_{15}H_{21}ClN_2O_6S$ : 392,85 - Calculé C : 45,9 ; H: 5,4 ; N : 7,1 - Trouvé C : 45,7; H : 5,3 ; N : 7,2.

EXEMPLE 9

[ (Chloro-5 diméthoxy-2,4) benzènesulfonyl ]-1 pipérazine (composé n° 1 220)

11,78 g (0,03 mole) de carbéthoxy-4 [ (chloro-5 diméthoxy-2,4) benzènesulfonyl ]-1 pipérazine sont mis en solution dans 40 g de potasse, dans 100 ml d'eau et 20 ml d'éthanol, puis portés au reflux pendant 8 heures. Après refroidissement et élimination sous vide de l'éthanol, la phase aqueuse est extraite par deux fois 100 ml d'acétate d'éthyle. La phase organique est séchée sur sulfate de sodium puis évaporée à sec sous vide. Les cristaux sont recristallisés dans l'acétonitrile.

On a obtenu 6,44 g de produit.

Rendement : 67 % - F° : 148°C

Analyse $C_{12}H_{17}ClN_2O_4S$ : 320,79 - Calculé C : 44,9 ; H : 5,3 ; N : 8,7 - Trouvé C : 44,8 ; H : 5,3 ; N : 8,7.

Les produits suivants ont été préparés selon les exemples 8 et 9 :

Composés n° 1 220, 1 241, 1 243.

EXEMPLE 10

[ (Diméthoxy-2,4 méthylsulfonyl-5) benzènesulfonyl ]-1 pipérazine (composé n° 1 242)

4,54 g (0,01 mole) de benzyl-4 [ (diméthoxy-2,4 méthylsulfonyl-5) benzènesulfonyl ]-1 pipérazine sont mis en solution dans 150 ml d'éthanol absolu et hydrogénés à pression ordinaire en présence de 0,5 g de palladium sur charbon à 50°C, pendant 3 heures. Après l'absorption théorique d'hydrogène, le catalyseur est éliminé par filtration. La phase éthanolique évaporée conduit à 4 g de cristaux qui peuvent être recristallisés dans le méthanol.

On a obtenu 4 g de produit.

Rendement : 95 % - F° : 140°C

Analyse $C_{13}H_{20}N_2O_6S_2$ : 364,43 - Calculé C : 42,8 ; H : 5,5 ; N : 7,7 - Trouvé C : 42,4 ; H : 5,4 ; N : 7,5.

Les produits suivants ont été préparés selon

l'exemple 10 :

Composés n° 1 220, 1 243, 1 204.

EXEMPLE 11

(Fluoro-4' butyrophénone)-4 [ (chloro-5 diméthoxy-2,4 benzènesulfonyl ]-1 pipérazine (composé n° 1 251)

A 4,8 g (0,015 mole) de [ (chloro-5 diméthoxy-2,4) benzènesulfonyl ]-1 pipérazine dans 80 ml de méthylisobutylcétone anhydre sont ajoutés 10 g de carbonate de potassium anhydre, puis goutte à goutte, 8,03 g (0,040 mole) de chloro p.fluorobutyrophénone. Le milieu réactionnel est porté au reflux pendant 48 heures. Après évaporation sous vide, le résidu est repris dans 100 ml d'eau, puis extrait par deux fois 100 ml d'acétate d'éthyle.

La phase organique séchée sur sulfate de sodium est évaporée à sec sous vide, le résidu est chromatographié sur silice éluant chloroforme pour donner 4 g de produit qui est recristallisé dans l'éthanol.

On a obtenu 1,88 g de produit.

Rendement : 26 % - F° : 120°C

Analyse $C_{22}H_{26}ClFN_2O_5S$ : 484,97 - Calculé C : 54,5 ; H : 5,4 ; N : 5,8 - Trouvé C : 54,5 ; H : 5,4 ; N : 5,6.

De la même façon, on prépare la (fluoro-4' butyrophénone)-4 [ (chloro-5 méthoxy-2) benzènesulfonyl ]-1 pipérazine.

EXEMPLE 12

Benzoyl-4 [ (chloro-5 diméthoxy-2,4) benzènesulfonyl ]-1 pipérazine (composé n° 1 272)

A 4,8 g (0,015 mole) de [ (chloro-5 diméthoxy-2,4) benzènesulfonyl ]-1 pipérazine dans 40 ml de chlorure de méthylène anhydre, on ajoute, à 0°C et sous agitation, 10 ml de triéthylamine redistillée puis 2,11 g (0,015 mole) de chlorure de benzoyle redistillé, goutte à goutte, en solution dans 10 ml de chlorure de méthylène. L'addition terminée, le milieu réactionnel

est ramené à température ambiante et laissé sous agitation 12 heures.

Après évaporation à sec sous vide, le résidu est repris dans 20 ml d'eau, les cristaux sont essorés, lavés à l'eau, séchés puis recristallisés dans l'éthanol.

On a obtenu 4,22 g de produit.

Rendement : 66 % - F° : 210°C

Analyse $C_{19}H_{21}ClN_2O_5S$ : 424,89 - Calculé C : 53,7 ; H : 5,0 ; N : 6,6 - Trouvé C : 53,7 ; H : 5,0 ; N : 6,6.

Les produits suivants ont été préparés selon l'exemple 12 :

- avec le chlorure de benzoyle :

Composés n° 1 273, 1 308, 1 392, 1 393, 1 394, 1 502 ;

- avec le chlorure d'adamantoyle-1 :

Composés n° 1 270, 1 271, 1 305, 1 307, 1 395, 1 404, 1 406, 1 407, 1 503 ;

- avec le chlorure de furoyle-2 :

Composés n° 1 274, 1 275, 1 304, 1 306, 1 396, 1 402, 1 403, 1 405.

EXEMPLE 13

(Dihydroxy-2,3 propyl)-4 [(ditrifluorométhyl-3,5) benzènesulfonyl]-1 homopipérazine (composé n° 1 488)

A 3,76 g (0,01 mole) de [(ditrifluorométhyl-3,5) benzènesulfonyl]-1 homopipérazine dans 30 ml d'acétonitrile, on ajoute sous agitation 0,74 g (0,01 mole) d'époxy-2,3 propanol-1 en solution dans 5 ml d'acétonitrile. Le milieu réactionnel est porté au reflux pendant 8 heures. Après évaporation à sec sous vide, on obtient un résidu cristallin qui est chromatographié sur colonne de silice éluant chloroforme 90-méthanol 10. Le produit est recristallisé dans l'acétate d'éthyle.

On a obtenu 2,2 g de produit.

Rendement : 49 % - F° : 128°C

Analyse $C_{16}H_{20}F_6N_2O_4S$ : 450,414 - Calculé C : 42,7 ; H : 4,5 ; N : 6,2 - Trouvé C : 42,5 ; H : 4,4 ; N : 6,4.

De la même façon, on prépare la (dihydroxy-2,3 propyl)-4 [ (ditrifluorométhyl-3,5) benzènesulfonyl ]-1 pipérazine (composé n° 1 397).

EXEMPLE 14

[ (Amino-4 méthoxy-2) benzènesulfonyl ]-1 méthyl-4 pipérazine (composé n° 1 425)

4,6 g (0,015 mole) de [ (méthoxy-2 nitro-4) benzènesulfonyl ]-1 méthyl-4 pipérazine en solution dans 200 ml d'éthanol anhydre sont hydrogénés à 20°C et à pression ordinaire en présence de nickel de Raney. Après absorption théorique d'hydrogène, le catalyseur est éliminé par filtration.

Le filtrat évaporé à sec donne un résidu cristallin. Le produit est recristallisé dans le minimum d'éthanol.

On a obtenu 4 g de produit.

Rendement : 93 % - F° : 174°C

Analyse $C_{12}H_{19}N_3O_3S$ : 285,369 - Calculé C : 50,5 ; H : 6,7 ; N : 14,7 - Trouvé C : 50,4 ; H : 6,6 ; N : 14,8.

De la même façon, on prépare l'[ (amino-4 chloro-5 méthoxy-2) benzènesulfonyl ]-1 méthyl-4 pipérazine (composé n° 1 427).

EXEMPLE 15

[ (Benzamido-4 méthoxy-2) benzènesulfonyl ]-1 méthyl-4 pipérazine (composé n° 1 426)

A 1,42 g (0,005 mole) d'[ (amino-4 méthoxy-2) benzènesulfonyl ]-1 méthyl-4 pipérazine dans 10 ml de chlorure de méthylène anhydre sont ajoutés à 0°C et sous agitation, 4 ml de triéthylamine, puis 0,8 g de chlorure de benzoyle en solution dans 5 ml de chlorure de méthylène anhydre.

Le milieu réactionnel ramené à température ambiante est agité pendant 12 heures puis évaporé à sec sous vide. Le résidu est repris dans 10 ml d'eau, puis

extrait par 2 x 25 ml d'acétate d'éthyle. La phase organique séchée sur $Na_2SO_4$, puis évaporée sous vide donne un résidu solide qui est purifié par chromatographie sur silice éluant ($CHCl_3$ 95, MeOH 5).

Le produit est recristallisé dans l'acétate d'éthyle.

F° : 147°C

Analyse $C_{19}H_{23}N_3O_4S$ : 389,478 - Calculé C : 58,6 ; H : 5,9 ; N : 10,8 - Trouvé C : 58,3 ; H : 5,9 ; N : 10,5.

EXEMPLE 16

[ (Acétamido-4 chloro-5 méthoxy-2) benzènesulfonyl ]-1 méthyl-4 pipérazine (composé n° 1 428)

1,6 g (0,005 mole) d' [ (amino-4 chloro-5 méthoxy-2) benzènesulfonyl ]-1 méthyl-4 pipérazine dans 3 ml d'anhydride acétique distillé et une goutte d'acide sulfurique concentré sont portés au reflux pendant 3 heures. Après refroidissement, on ajoute 10 ml d'eau, puis de la soude 2N pour amener à pH 10. On extrait par deux fois 50 ml d'acétate d'éthyle. La phase organique est séchée sur sulfate de sodium, puis évaporée à sec sous vide. Le résidu est recristallisé dans l'acétate d'éthyle.

F° : 172°C

Analyse $C_{14}H_{20}ClN_3O_4S$ : 361,856 - Calculé C : 46,5 ; H : 5,6 ; N : 11,6 - Trouvé C : 46,2 ; H : 5,4 ; N : 11,5

Les produits des exemples 4 à 16 ainsi que les produits préparés selon les exemples 4 à 16, mentionnés ci-dessus, sont des produits industriels nouveaux.

Le tableau I ci-après indique pour chacun des composés selon l'invention et des composés qui entrent dans la préparation des médicaments selon l'invention et qui ont été préparés comme il a été indique ci-dessus la formule, le poids moléculaire, le point de fusion et le rendement.

TABLEAU I

| COMPOSE N° | V | W | X | Y | n | Z | FORMULE BRUTE | P.M. | F°C | Rdt % |
|---|---|---|---|---|---|---|---|---|---|---|
| 1202 | $OCH_3$ | H | $OCH_3$ | $OCH_3$ | 2 | O | $C_{13}H_{19}NO_6S$ | 317,37 | 104 | 81 |
| 1209 | $OCH_3$ | H | $NO_2$ | Cl | 2 | O | $C_{11}H_{13}ClN_2O_6S$ | 336,75 | 179 | 66 |
| 1215 | $OCH_3$ | H | $OCH_3$ | $SO_2$-$iC_3H_7$ | 2 | O | $C_{15}H_{23}NO_7S_2$ | 393,47 | 226 | 60 |
| 1216 | $OCH_3$ | H | $OCH_3$ | Br | 2 | O | $C_{12}H_{16}BrNO_5S$ | 366,23 | 186 | 76 |
| 1223 | $OCH_3$ | H | $OCH_3$ | Cl | 2 | O | $C_{12}H_{16}ClNO_5S$ | 321,77 | 186 | 74 |
| 1246 | $OCH_3$ | H | $OCH_3$ | H | 2 | O | $C_{12}H_{17}NO_5S$ | 287,33 | 124 | 83 |
| 1247 | $OCH_3$ | H | H | Cl | 2 | O | $C_{11}H_{14}ClNO_4S$ | 291,75 | 136 | 89 |
| 1208 | $OCH_3$ | H | $NO_2$ | Cl | 2 | $CH_2$ | $C_{12}H_{15}ClN_2O_5S$ | 334,77 | 184 | 74 |
| 1211 | $OCH_3$ | H | $OCH_3$ | $SO_2$-$CH_3$ | 2 | $CH_2$ | $C_{14}H_{21}NO_6S_2$ | 363,45 | 190 | 73 |
| 1218 | $OCH_3$ | H | $OCH_3$ | Br | 2 | $CH_2$ | $C_{13}H_{18}BrNO_4S$ | 364,26 | 149 | 77 |
| 1224 | $OCH_3$ | H | $OCH_3$ | Cl | 2 | $CH_2$ | $C_{13}H_{18}ClNO_4S$ | 319,80 | 152 | 86 |

| COMPOSE N° | V | W | X | Y | n | Z | FORMULE BRUTE | P.M. | F°C | Rdt % |
|---|---|---|---|---|---|---|---|---|---|---|
| 1248 | $OCH_3$ | H | $OCH_3$ | H | 2 | $CH_2$ | $C_{13}H_{19}NO_4S$ | 285,36 | 105 | 86 |
| 1249 | $OCH_3$ | H | H | Cl | 2 | $CH_2$ | $C_{12}H_{16}ClNO_3S$ | 289,78 | 86 | 86 |
| 1204 | $OCH_3$ | H | $OCH_3$ | H | 2 | NH | $C_{12}H_{16}N_2O_4S$, HBr | 365,25 | 102 | '99 |
| 1220 | $OCH_3$ | H | $OCH_3$ | Cl | 2 | NH | $C_{12}H_{17}ClN_2O_4S$ | 320,79 | 149 | 65 |
| 1240 | $OCH_3$ | H | H | Cl | 2 | NH | $C_{11}H_{15}ClN_2O_3S$ | 290,76 | 145 | 67 |
| 1241 | $OCH_3$ | H | $NO_2$ | Cl | 2 | NH | $C_{11}H_{14}ClN_3O_5S$ | 335,76 | 162 | 51 |
| 1242 | $OCH_3$ | H | $OCH_3$ | $SO_2-CH_3$ | 2 | NH | $C_{13}H_{20}N_2O_6S_2$ | 364,43 | 140 | 95 |
| 1243 | $OCH_3$ | H | $OCH_3$ | $OCH_3$ | 2 | NH | $C_{13}H_{20}N_2O_5S$ | 316,37 | 108 | 31 |
| 1400 | H | $CF_3$ | H | $CF_3$ | 2 | NH | $C_{12}H_{12}F_6N_2O_2S$, HCl | 416,77 | 105 | 68 |
| 1401 | H | $CF_3$ | Cl | H | 2 | NH | $C_{11}H_{12}ClF_3N_2O_2S$ | 328,75 | 118 | 76 |
| 1301 | $OCH_3$ | H | $OCH_3$ | $OCH_3$ | 3 | NH | $C_{14}H_{22}N_2O_5S$ | 330,40 | 99 | 50 |

| COMPOSE N° | V | W | X | Y | n | Z | FORMULE BRUTE | P.M. | F°C | Rdt % |
|---|---|---|---|---|---|---|---|---|---|---|
| 1302 | $OCH_3$ | H | $OCH_3$ | $SO_2-CH_3$ | 3 | NH | $C_{14}H_{22}N_2O_6S_2$ | 378,47 | 154 | 49 |
| 1303 | $OCH_3$ | H | $OCH_3$ | Cl | 3 | NH | $C_{13}H_{19}ClN_2O_4S$ | 334,83 | 138 | 78 |
| 1408 | H | $CF_3$ | H | $CF_3$ | 3 | NH | $C_{13}H_{14}F_6N_2O_2S$ | 376,33 | 89 | 78 |
| 1399 | H | $CF_3$ | Cl | H | 3 | NH | $C_{12}H_{14}ClF_3N_2O_2S$, HCl | 379,24 | 256 | 46 |
| 1260 | $OCH_3$ | H | $OCH_3$ | $OCH_3$ | 2 | $N-CH_3$ | $C_{14}H_{22}N_2O_5S$ | 330,40 | 114 | 66 |
| 1261 | $OCH_3$ | H | $OCH_3$ | Cl | 2 | $N-CH_3$ | $C_{13}H_{19}ClN_2O_4S$ | 334,82 | 131 | 70 |
| 1292 | $OCH_3$ | H | $NO_2$ | Cl | 2 | $N-CH_3$ | $C_{12}H_{16}ClN_3O_5S$ | 349,79 | 135 | 82 |
| 1263 | $OCH_3$ | H | H | Cl | 2 | $N-CH_2-CH_2OH$ | $C_{13}H_{19}ClN_2O_4S$ | 334,82 | 111 | 72 |
| 1264 | $OCH_3$ | H | $OCH_3$ | $OCH_3$ | 2 | $N-CH_2-CH_2OH$ | $C_{15}H_{24}N_2O_6S$ | 360,43 | 103 | 65 |
| 1290 | $OCH_3$ | H | $NO_2$ | Cl | 2 | $N-CH_2-CH_2OH$ | $C_{13}H_{18}ClN_3O_6S$ | 379,82 | 166 | 51 |
| 1294 | $OCH_3$ | H | $OCH_3$ | $SO_2-CH_3$ | 2 | $N-CH_2-CH_2OH$ | $C_{15}H_{24}N_2O_7S_2$ | 408,50 | 122 | 72 |

| COMPOSE N° | V | W | X | Y | n | Z | FORMULE BRUTE | P.M. | F°C | Rdt % |
|---|---|---|---|---|---|---|---|---|---|---|
| 1296 | $OCH_3$ | H | $OCH_3$ | Cl | 2 | $N-CH_2-CH_2OH$ | $C_{14}H_{21}ClN_2O_5S$ | 364,86 | 118 | 74 |
| 1389 | H | $CF_3$ | H | $CF_3$ | 2 | $N-CH_2-CH_2OH$ | $C_{14}H_{16}F_6N_2O_3S$ | 406,34 | 84 | 41 |
| 1398 | H | $CF_3$ | Cl | H | 2 | $N-CH_2-CH_2OH$ | $C_{13}H_{16}ClF_3N_2O_3S$ | 372,79 | 87 | 55 |
| 1262 | $OCH_3$ | H | H | Cl | 2 | $N-CH_2-CHOH-CH_2OH$ | $C_{14}H_{21}ClN_2O_5S$ | 364,85 | 115 | 77 |
| 1265 | $OCH_3$ | H | $OCH_3$ | $OCH_3$ | 2 | $N-CH_2-CHOH-CH_2OH$ | $C_{16}H_{26}N_2O_7S$ | 390,46 | 158 | 62 |
| 1291 | $OCH_3$ | H | $NO_2$ | Cl | 2 | $N-CH_2-CHOH-CH_2OH$ | $C_{14}H_{20}ClN_3O_7S$ | 409,68 | 174 | 33 |
| 1293 | $OCH_3$ | H | $OCH_3$ | $SO_2-CH_3$ | 2 | $N-CH_2-CHOH-CH_2OH$ | $C_{16}H_{26}N_2O_8S_2$ | 434,52 | 205 | 31 |
| 1295 | $OCH_3$ | H | $OCH_3$ | Cl | 2 | $N-CH_2-CHOH-CH_2OH$ | $C_{15}H_{23}ClN_2O_6S$ | 394,89 | 118 | 69 |
| 1334 | H | $CF_3$ | H | H | 2 | $N-CH_2-CHOH-CH_2OH$ | $C_{14}H_{19}F_3N_2O_4S$, $CH_3SO_3H$ | 464,49 | 200 | 43 |
| 1387 | H | $CF_3$ | Cl | H | 2 | $N-CH_2-CHOH-CH_2OH$ | $C_{14}H_{18}ClF_3N_2O_4S$ | 402,82 | 119 | 41 |
| 1397 | H | $CF_3$ | H | $CF_3$ | 2 | $N-CH_2-CHOH-CH_2OH$ | $C_{15}H_{18}F_6N_2O_4S$ | 436,37 | 104 | 31 |

| COMPOSE N° | V | W | X | Y | n | Z | FORMULE BRUTE | P.M. | F°C | Rdt % |
|---|---|---|---|---|---|---|---|---|---|---|
| 1206 | $OCH_3$ | H | $NO_2$ | Cl | 2 | $N-CH_2-C_6H_5$ | $C_{18}H_{20}ClN_3O_5S$ | 425,89 | 140 | 45 |
| 1213 | $OCH_3$ | H | $OCH_3$ | $SO_2-CH_3$ | 2 | $N-CH_2-C_6H_5$ | $C_{20}H_{26}N_2O_6S_2$ | 454,55 | 165 | 59 |
| 1214 | $OCH_3$ | H | $OCH_3$ | $SO_2-iC_3H_7$ | 2 | $N-CH_2-C_6H_5$ | $C_{22}H_{30}N_2O_6S_2$ | 482,60 | 180 | 74 |
| 1219 | $OCH_3$ | H | $OCH_3$ | Br | 2 | $N-CH_2-C_6H_5$ | $C_{19}H_{23}BrN_2O_4S$ | 455,37 | 164 | 73 |
| 1222 | $OCH_3$ | H | $OCH_3$ | Cl | 2 | $N-CH_2-C_6H_5$ | $C_{19}H_{23}ClN_2O_4S$ | 410,92 | 150 | 76 |
| 1244 | $OCH_3$ | H | $OCH_3$ | H | 2 | $N-CH_2-C_6H_5$ | $C_{19}H_{24}N_2O_4S$ | 376,47 | 138 | 89 |
| 1245 | $OCH_3$ | H | H | Cl | 2 | $N-CH_2-C_6H_5$ | $C_{18}H_{21}ClN_2O_3S$ | 380,89 | 104 | 46 |
| 1335 | H | $CF_3$ | H | H | 2 | $N-CH_2-C_6H_5$ | $C_{18}H_{19}F_3N_2O_2S$, HCl | 420,89 | 250 | 83 |
| 1390 | H | $CF_3$ | H | $CF_3$ | 2 | $N-CH_2-C_6H_5$ | $C_{19}H_{18}F_6N_2O_2S$ | 452,43 | 85 | 67 |
| 1391 | H | $CF_3$ | Cl | H | 2 | $N-CH_2-C_6H_5$ | $C_{18}H_{18}ClF_3N_2O_2S$ | 418,88 | 124 | 63 |
| 1251 | $OCH_3$ | H | $OCH_3$ | Cl | 2 | $N-(CH_2)_3-CO-$ 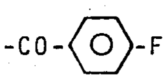 -F | $C_{22}H_{26}ClFN_2O_5S$ | 484,97 | 120 | 26 |

| COMPOSE N° | V | W | X | Y | n | Z | FORMULE BRUTE | P.M. | F°C | Rdt % |
|---|---|---|---|---|---|---|---|---|---|---|
| 1269 | $OCH_3$ | H | H | Cl | 2 | $N-(CH_2)_3-CO-$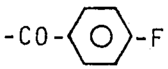-F | $C_{21}H_{24}ClFN_2O_4S$, HCl | 491,41 | 190 | 10 |
| 1297 | $OCH_3$ | H | $OCH_3$ | $OCH_3$ | 2 | N-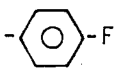-F | $C_{19}H_{23}FN_2O_5S$ | 410,47 | 188 | 83 |
| 1298 | $OCH_3$ | H | $OCH_3$ | $SO_2-CH_3$ | 2 | N-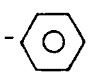-F | $C_{19}H_{23}FN_2O_6S_2$ | 458,53 | 255 | 71 |
| 1299 | $OCH_3$ | H | H | Br | 2 | N-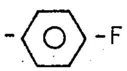-F | $C_{17}H_{18}BrFN_2O_3S$ | 429,32 | 125 | 58 |
| 1300 | $OCH_3$ | H | $OCH_3$ | Cl | 2 | N-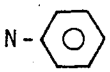-F | $C_{18}H_{20}ClFN_2O_4S$ | 414;90 | 170 | 64 |
| 1336 | H | $CF_3$ | H | H | 2 | N-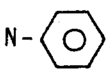-F | $C_{17}H_{16}F_4N_2O_2S$ | 388,39 | 100 | 75 |
| 1353 | $OCH_3$ | H | H | $NO_2$ | 2 | N-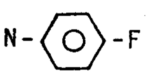-F | $C_{17}H_{18}FN_3O_5S$ | 395,42 | 205 | 73 |
| 1388 | H | $CF_3$ | H | $CF_3$ | 2 | N-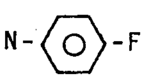-F | $C_{18}H_{15}FN_2O_2S$ | 456,40 | 152 | 62 |
| | | | | | | | | | | |
| 1272 | $OCH_3$ | H | $OCH_3$ | Cl | 2 | N-CO-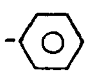 | $C_{19}H_{21}ClN_2O_5S$ | 424,89 | 210 | 66 |
| 1273 | $OCH_3$ | H | $OCH_3$ | H | 2 | N-CO-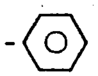 | $C_{19}H_{22}N_2O_5S$ | 390,45 | 171 | 68 |

| COMPOSE N° | V | W | X | Y | n | Z | FORMULE BRUTE | P.M. | F°C | Rdt % |
|---|---|---|---|---|---|---|---|---|---|---|
| 1392 | H | $CF_3$ | H | $CF_3$ | 2 | N-CO- 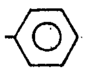 | $C_{19}H_{16}F_6N_2O_3S$ | 466,42 | 164 | 45 |
| 1308 | $OCH_3$ | H | $OCH_3$ | Cl | 3 | N-CO- 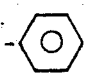 | $C_{20}H_{23}ClN_2O_5S$ | 438,94 | 210 | 60 |
| 1393 | H | $CF_3$ | Cl | H | 3 | N-CO- 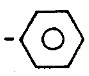 | $C_{19}H_{18}ClF_3N_2O_3S$ | 446,89 | 95 | 76 |
| 1394 | H | $CF_3$ | H | $CF_3$ | 3 | N-CO- 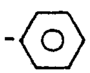 | $C_{20}H_{18}F_6N_2O_3S$ | 480,44 | 120 | 35 |
| 1274 | $OCH_3$ | H | $OCH_3$ | Cl | 2 | N-CO- 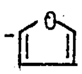 | $C_{17}H_{19}ClN_2O_6S$ | 414,86 | 138 | 81 |
| 1275 | $OCH_3$ | H | H | Cl | 2 | N-CO- 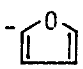 | $C_{16}H_{17}ClN_2O_5S$ | 384,82 | 131 | 56 |
| 1402 | H | $CF_3$ | Cl | H | 2 | N-CO- 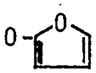 | $C_{16}H_{14}ClF_3N_2O_4S$ | 422,82 | 163 | 71 |
| 1403 | H | $CF_3$ | H | $CF_3$ | 2 | N-CO- 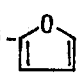 | $C_{17}H_{14}F_6N_2O_4S$ | 456,38 | 138 | 62 |
| 1304 | $OCH_3$ | H | $OCH_3$ | $OCH_3$ | 3 | N-CO- 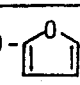 | $C_{19}H_{24}N_2O_7S$ | 424,47 | 175 | 75 |
| 1306 | $OCH_3$ | H | $OCH_3$ | $SO_2-CH_3$ | 3 | N-CO- 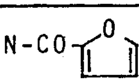 | $C_{19}H_{24}N_2O_8S_2$ | 472,53 | 187 | 73 |

0138720

| COMPOSE N° | V | W | X | Y | n | Z | FORMULE BRUTE | P.M. | F°C | Rdt % |
|---|---|---|---|---|---|---|---|---|---|---|
| 1396 | H | $CF_3$ | H | $CF_3$ | 3 | N-CO- 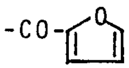 | $C_{18}H_{16}F_6N_2O_4S$ | 470,40 | 130 | 34 |
| 1405 | H | $CF_3$ | Cl | H | 3 | N-CO- 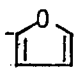 | $C_{17}H_{16}ClF_3N_2O_4S$ | 436,85 | 104 | 75 |
| 1270 | $OCH_3$ | H | $OCH_3$ | Cl | 2 | N-CO- 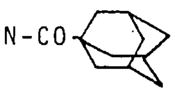 | $C_{23}H_{31}ClN_2O_5S$ | 483,02 | 266 | 88 |
| 1271 | $OCH_3$ | H | $OCH_3$ | H | 2 | N-CO- 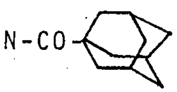 | $C_{23}H_{32}N_2O_5S$ | 448,58 | 188 | 60 |
| 1404 | H | $CF_3$ | H | $CF_3$ | 2 | N-CO- 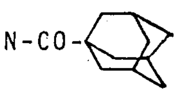 | $C_{23}H_{26}F_6N_2O_3S$ | 524,54 | 183 | 54 |
| 1406 | H | $CF_3$ | Cl | H | 2 | N-CO- 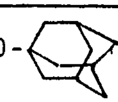 | $C_{22}H_{26}ClF_3N_2O_3S$ | 490,99 | 118 | 57 |
| 1305 | $OCH_3$ | H | $OCH_3$ | $OCH_3$ | 3 | N-CO- 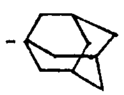 | $C_{25}H_{36}N_2O_6S$ | 492,63 | 162 | 61 |
| 1307 | $OCH_3$ | H | $OCH_3$ | $SO_2-CH_3$ | 3 | N-CO- 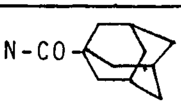 | $C_{24}H_{33}ClN_2O_5S$ | 497,05 | 169 | 60 |
| 1395 | H | $CF_3$ | H | $CF_3$ | 3 | N-CO- 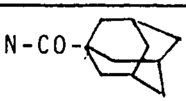 | $C_{24}H_{28}F_6N_2O_3S$ | 538,57 | 148 | 27 |
| 1407 | H | $CF_3$ | Cl | H | 3 | N-CO- 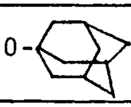 | $C_{23}H_{28}ClF_3N_2O_3S$ | 505,01 | 145 | 57 |

| COMPOSE N° | V | W | X | Y | n | Z | FORMULE BRUTE | P.M. | F°C | Pdt % |
|---|---|---|---|---|---|---|---|---|---|---|
| 1424 | $OCH_3$ | H | $NO_2$ | H | 2 | $N-CH_3$ | $C_{12}H_{17}N_3O_5S$ | 315,35 | 110 | 72 |
| 1425 | $OCH_3$ | H | $NH_2$ | H | 2 | $N-CH_3$ | $C_{12}H_{19}N_3O_3S$ | 285,37 | 174 | 93 |
| 1426 | $OCH_3$ | H | $NH-CO-C_6H_5$ | H | 2 | $N-CH_3$ | $C_{19}H_{23}N_3O_4S$ | 389,48 | 147 | 40 |
| 1427 | $OCH_3$ | H | $NH_2$ | Cl | 2 | $N-CH_3$ | $C_{12}H_{18}ClN_3O_3S$ | 319,81 | 162 | 90 |
| 1428 | $OCH_3$ | H | $NHCOCH_3$ | Cl | 2 | $N-CH_3$ | $C_{14}H_{20}ClN_3O_4S$ | 361,86 | 172 | 30 |
| | | | | | | | | | | |
| | | | | | | | | | | |
| | | | | | | | | | | |
| | | | | | | | | | | |
| | | | | | | | | | | |
| | | | | | | | | | | |

| Composé n° | V | W | X | Y | n | Z | Formule brute | P.M. | F | Rdt |
|---|---|---|---|---|---|---|---|---|---|---|
| 1488 | H | $CF_3$ | H | $CF_3$ | 3 | $NCH_2CHOHCH_2OH$ | $C_{16}H_{20}F_6N_2O_4S$ | 450,414 | 128 | 48 |
| 1500 | H | $CF_3$ | H | H | 2 | $NCH_2CH_2CH_2OH$ | $C_{14}H_{19}F_3N_2O_3S$ | 352,375 | 87 | 30 |
| 1502 | H | $CF_3$ | H | H | 2 | 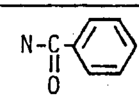 | $C_{18}H_{17}F_3N_2O_3S$ | 398,403 | 118 | 65 |
| 1503 | H | $CF_3$ | H | H | 2 | 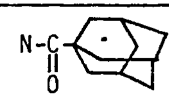 | $C_{22}H_{27}F_3N_2O_3S$ | 456,527 | 170 | 61 |
| 1504 | H | $CF_3$ | H | H | 2 | NH | $C_{11}H_{13}F_3N_2O_2S$ | 294,295 | 65 | 73 |
| 1505 | H | $CF_3$ | H | H | 2 | $N-CH_2CH_2OH$ | $C_{13}H_{17}F_3N_2O_3S$ | 338,348 | 95 | 82 |
| 1521 | H | $CF_3$ | H | H | 3 | NH | $C_{12}H_{15}F_3N_2O_2S$ | 308,330 | 110 | 40 |

Les composés chimiques ci-dessus définis ainsi que leurs sels organiques ou minéraux physiologiquement acceptables peuvent entrer, à titre de substances actives, dans la préparation de médicaments présentant un ensemble de propriétés thérapeutiques et pharmacologiques de grande valeur.

Les médicaments selon l'invention exercent une action sur le système nerveux central, notamment anxiolytique.

Ces propriétés pharmacologiques ont été étudiées en prenant comme substance de référence la trimétozine (commercialisée sous la marque Opalène).

Les médicaments selon l'invention sont avantageusement utilisés dans le traitement des maladies névrotiques, avec anxiété, irritabilité, troubles du caractère.

Les composés qui entrent dans les médicaments selon l'invention sont à cet effet conditionnés avec les excipients et adjuvants traditionnels, notamment ceux utilisés pour l'administration par voie orale telle que comprimés, poudres, gélules, ampoules buvables ou pour l'administration sous forme de solutions injectables, suppositoires.

L'administration des médicaments contenant les composés selon l'invention est effectuée de préférence par voie orale et les doses de composés administrés sont de préférence comprises entre 1 et 1 000 mg, et notamment entre 5 et 250 mg/jour, selon le mode d'administration.

Un exemple de préparation pharmaceutique des produits selon l'invention est constitué par un comprimé ou gélule contenant 5 ou 10 mg de l'un au moins des produits définis ci-dessus.

A titre d'exemples, différents essais pour la mise en oeuvre des propriétés pharmacologiques des

composés selon l'invention sont rapportés ci-après.

ETUDE DE LA TOXICITE

Les résultats relatifs à la toxicité sont rapportés ci-après dans le tableau II suivant.

0138720

TABLEAU II

TOXICITE AIGUE

| Composés n° | Voie | Doses mg/kg | | | Nombre de doses | Nombre de souris |
|---|---|---|---|---|---|---|
| | | $DL_0$ | $DL_{50}$ | $DL_{100}$ | | |
| 1 243 | IV | 200 | 200 | 325 | 8 | 80 |
| 1 292 | IP | 400 | 650 | 1 000 | 7 | 70 |
| 1 295 | IP | 600 | 820 | 1 000 | 9 | 90 |
| 1 245 | IP | > 2 000 | > 2 000 | > 2 000 | 3 | 30 |
| 1 298 | IP | > 2 000 | > 2 000 | > 2 000 | 3 | 30 |

Pour les composés n° 1 393 et 1 402, la $DL_0$ est supérieure à 2 000 mg/kg par voie orale.

Pour les composés n° 1 400 et 1 427, la $DL_0$ est respectivement de l'ordre de 250 et 500 mg/kg, tandis que la $DL_{50}$ est respectivement d'environ 700 et d'environ 800 mg/kg.

Les composés suivants : n° 1 393, 1 400, 1 402, 1 427, 1 243, 1 292, 1 295, 1 245, 1 298 ont montré que le rapport entre la dose efficace et la toxicité est satisfaisant, ce qui doit conduire à un indice thérapeutique satisfaisant.

ETUDE DES PROPRIETES DES COMPOSES SELON L'INVENTION DANS LE TEST DES 4 PLAQUES

Le test des 4 plaques ou épreuve de conditionnement suppressif a été décrit par SLOTNIK et JARVID (Science, 1966, 154, 1 207-1 208) en 1968, puis développé par ARON-SAMUEL (Thèse Doctorat Etat Médecine, Paris 1970).

Cet auteur a étudié sur le test des substances appartenant aux différentes familles de psychotropes et seuls les tranquillisants (tranquillisants mineurs) ont affecté le comportement des souris aux doses inactives sur la motilité. Selon SIMON et COLONNA, il s'agit d'une excellente méthode de tri des substances tranquillisantes ("Mieux connaître et mieux prescrire les psychotropes, PIL Ed. Paris, 1972).

Matériel et méthode

Principe actif

La méthode est basée sur l'atténuation ou la suppression de l'état anxieux induit par chocs électriques lors du déplacement des animaux (souris) dans une enceinte spéciale.

L'effet anxiolytique est matérialisé par une augmentation des déplacements aux doses n'affectant pas la motilité.

Animaux

Souris mâles Swiss en provenance du CERJ (53680 Le Genest) d'un poids corporel de 20-22 g et acclimatées 8 jours à l'animalerie du laboratoire. Pour chaque dose, les lots sont de 15 animaux n'ayant jamais subi le test.

Matériel

L'appareil utilisé est de type Apelex.

Il se compose d'une boîte parallélépipédique en plastique blanc, munie d'un couvercle en plexiglas transparent, mobile grâce à deux charnières. Le plancher, rectangulaire, est constitué de quatre plaques en métal conducteur dont la distance entre elles et les parois de la boîte est de 0,5 cm.

Ces plaques sont reliées à un générateur d'impulsions Apelex. La différence de potentiel à vide, nulle entre deux plaques en diagonale, est de 180 volts entre deux plaques adjacentes. Un déclencheur permet d'envoyer une impulsion de courant électrique constant (de 0 à 2 milliampères) pendant un temps choisi (de 0,1 à 5 secondes).

Protocole

La manipulation se déroule dans un local silencieux à température constante, l'expérimentateur dominant la boîte de telle sorte qu'il puisse suivre tous les déplacements de la souris déposée sur une des plaques. Le déclencheur est éloigné de la boîte afin que son bruit ne modifie pas le comportement de l'animal.

Pour chaque animal, l'expérimentation est réalisée selon les modalités suivantes :

- exploration préliminaire de 15 secondes, sans choc électrique ;
- exploration punie (choc électrique à chaque déplacement entre deux plaques adjacentes) pendant 60 secondes.

Les chocs électriques ont une durée de 0,5 seconde et une intensité de 0,6 milliampère.

Dans chaque essai, les souris sont traitées par voie orale 60 minutes avant le test, soit par les substances pharmacologiques (composés selon l'invention n° 1 505, 1 260, 1 299, 1 503, 1 504, 1500, 1 406, 1 403, 1 408, 1 502, 1 243, 1 290, 1 294, 1 307, 1 405, 1 213, 1 263, 1 488) ou le solvant seul (lot témoin).

Les produits sont administrés à dose unique de 25 mg/kg, par voie orale, par tubage gastrique dans une solution de carboxyméthylcellulose à 3 %, 60 minutes avant le test.

Les témoins reçoivent uniquement la carboxyméthylcellulose.

EXPRESSION DES RESULTATS

Les résultats sont exprimés en pourcentage d'augmentation du nombre de passages punis par rapport au lot témoin (pourcentage d'augmentation).

RESULTATS

Ils sont rassemblés dans le tableau III suivant.

TABLEAU III

| Test des 4 plaques souris ♂ | % d'augmentation |
|---|---|
| 1505 | 35 |
| 1260 | 31 |
| 1299 | 28 |
| 1503 | 28 |
| 1504 | 26 |
| 1500 | 25 |
| 1406 | 24 |
| 1403 | 22 |
| 1408 | 22 |
| 1502 | 20 |
| 1243 | 18 |
| 1290 | 17 |
| 1294 | 17 |
| 1307 | 16 |
| 1405 | 14 |
| 1213 | 13 |
| 1263 | 10 |
| 1488 | 10 |

Les résultats de ces essais montrent l'activité anxiolytique des composés testés. Les composés de l'invention n° 1 505, 1 260, 1 299, 1 503, 1 504, 1 500, 1 406, 1 403 et 1 408 qui présentent un pourcentage d'augmentation supérieur à 20 % se révèlent particulièrement actifs.

Le test des quatre plaques a été également effectué sur une autre classe de composés selon l'invention, en utilisant le protocole indiqué ci-dessus et modifié comme suit.

Les souris sont traitées par voie orale 60 minutes avant le test, soit par les substances pharmacologiques (composés selon l'invention n° 1 407, 1 306, 1 426, 1 334, 1 425, 1 428, 1 301, 1 270, 1 265, 1 242, 1 224, 1 247, 1 304, 1 295, 1 211, 1 390, 1 396, 1 274, 1 389, 1 245, 1 427, 1 404, 1 302, 1 214, 1 264, 1 220, 1 400, 1 402 ou la trimétozine) ou le solvant seul (lot témoin).

Les produits sont administrés à dose unique de 5 mg/kg, par voie orale, par tubage gastrique dans une solution de carboxyméthylcellulose à 0,1 %, 60 minutes avant le test.

Les témoins reçoivent uniquement la carboxyméthylcellulose.

EXPRESSION DES RESULTATS

Les résultats sont exprimés par l'indice de variation du nombre de passages punis par rapport à la trimétozine utilisée comme référence (indice 100). L'indice 0 correspond au lot témoin.

RESULTATS

Les résultats obtenus sont rassemblés dans le tableau IV ci-après.

TABLEAU IV



| Test des 4 plaques souris ♂ | Indice de variation |
|---|---|
| 1407 | 179 |
| 1306 | 155 |
| 1426 | 154 |
| 1334 | 144 |
| 1425 | 133 |
| 1428 | 133 |
| 1301 | 120 |
| 1270 | 117 |
| 1265 | 107 |
| Trimétozine | 100 |
| 1242 | 97 |
| 1224 | 90 |
| 1247 | 90 |
| 1304 | 85 |
| 1295 | 70 |
| 1211 | 68 |
| 1390 | 64 |
| 1396 | 59 |
| 1274 | 53 |
| 1389 | 52 |
| 1245 | 48 |
| 1427 | 46 |
| 1404 | 43 |
| 1302 | 30 |
| 1214 | 29 |
| 1264 | 22 |
| 1220 | 20 |
| 1400 | 16 |
| 1402 | 16 |

Les résultats de ces essais confirment l'activité anxiolytique des composés de cette série.

Une classe préférée de composés est constituée par ceux qui présentent un indice de variation supérieur à 50, c'est-à-dire les composés n° 1 407, 1 306, 1 426, 1 334, 1 425, 1 428, 1 301, 1 270, 1 265, 1 242, 1 224, 1 247, 1 304, 1 295, 1 211, 1 390, 1 396, 1 274, 1 389.

REVENDICATIONS

1. Composés nouveaux répondant à la formule générale :

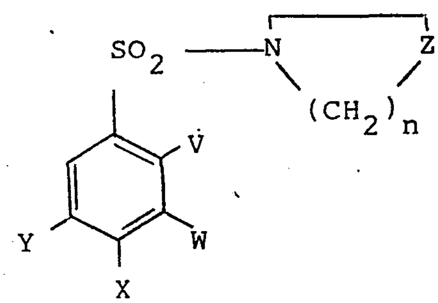

(I)

dans laquelle :

- V représente l'hydrogène, un radical $OR_1$ dans lequel $R_1$ représente un groupe alcoyle de 1 à 4 atomes de carbone ;

- W représente l'hydrogène, le groupe $CF_3$ ;

- X représente l'hydrogène, un halogène, le groupe $NO_2$, $NH_2$, un radical $NH-CO-R_2$ dans lequel $R_2$ représente un radical alcoyle de 1 à 4 atomes de carbone ou aryle, un radical $OR_1$ dans lequel $R_1$ représente un radical alcoyle de 1 à 4 atomes de carbone ;

- Y représente l'hydrogène, un halogène, le groupe $NO_2$, $NH_2$, $CF_3$, un radical $NH-CO-R_2$ dans lequel $R_2$ représente un radical alcoyle de 1 à 4 atomes de carbone ou aryle, un radical $OR_1$ dans lequel $R_1$ représente un radical alcoyle de 1 à 4 atomes de carbone, un radical $SO_2-R_3$ dans lequel $R_3$ représente un radical alcoyle de 1 à 4 atomes de carbone,

- n vaut 2 ou 3 ;

- Z représente un atome d'oxygène, le groupe $CH_2$ ou $NR_4$ dans lequel :

. $R_4$ représente :

- un hydrogène,

- un radical alcoyle inférieur de 1 à 6 atomes

de carbone,

- un radical hydroxyalcoyle de 1 à 3 atomes de carbone,

- un radical cycloalcanoyle de 3 à 10 atomes de carbone,

- un radical aryle non substitué ou substitué par un halogène, par le groupe $CF_3$, par un radical $OR_1$ dans lequel $R_1$ représente un radical alcoyle ayant de 1 à 4 atomes de carbone,

- un radical aralcoyle dans lequel la chaîne aliphatique présente de 1 à 4 atomes de carbone et peut comporter des groupes C = O et C-OH dans lequel le groupe aryle est non substitué ou substitué par un halogène, par le groupe $CF_3$, par un radical $OR_1$, dans lequel $R_1$ est un radical alcoyle ayant de 1 à 4 atomes de carbone,

- un radical aroyle ou ses isostères non substitués ou substitués par un halogène, par le groupe $CF_3$, par un radical $OR_1$ dans lequel $R_1$ est un groupe alcoyle de 1 à 4 atomes de carbone ;

sous réserve que, lorsque n = 2 :

- 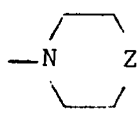 représente un groupe - 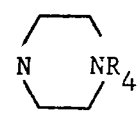

et

- • soit V représente $OR_1$, l'un au moins des éléments W, X ou Y étant différent de l'hydrogène,

•soit Y ou W représente $CF_3$.

2. Composés selon la revendication 1 répondant à la formule générale (II) suivante :

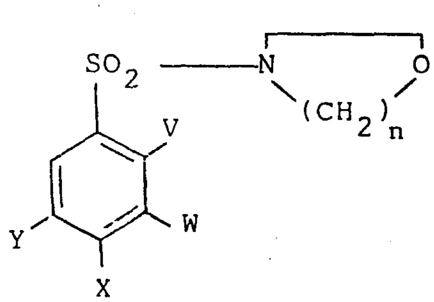

(II)

dans laquelle V, W, X, et Y ont les significations indiquées à la revendication 1 et n vaut 3.

3. Composés selon la revendication 1 répondant à la formule générale (III) suivante :

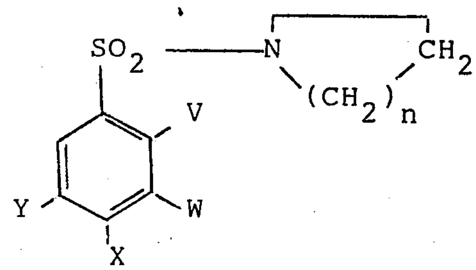

(III)

dans laquelle V, W, X, et Y ont les significations indiquées à la revendication 1 et n vaut 3.

4. Composés selon la revendication 1, répondant à la formule (IV) suivante :

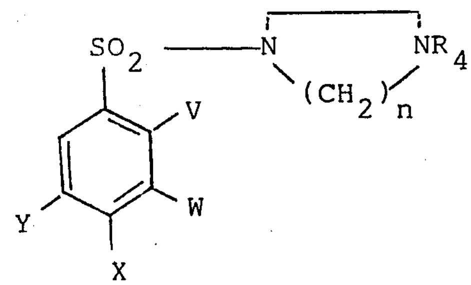

(IV)

dans laquelle V, W, X, Y ont les significations indiquées à la revendication 1 et $R_4$ représente un radical cyclopropanoyle, cyclopentanoyle, cyclohexanoyle, adamantoyle, benzoyle ou furoyle.

5. Composés selon la revendication 1 répondant à la formule générale (IV) suivante :

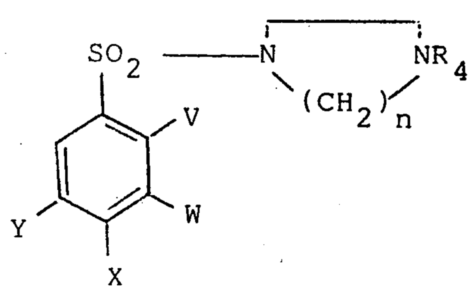

(IV)

dans laquelle V, W, Y et Y ont les significations indiquées à la revendication 1 et $R_4$ représente :

- l'hydrogène,
- un radical benzyle non substitué ou substitué par un halogène, par le groupe $CF_3$, par le radical $OR_1$, dans lequel $R_1$ est un alcoyle de 1 à 4 atomes de carbone,
- $CH_3$, $CH_2CH_2OH$, $CH_2CHOHCH_2OH$, $CH_2-C_6H_5$,

$(CH_2)_3-CO$ 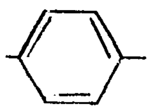 F, 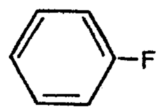 F,

6. Composés selon l'une quelconque des revendications 1 à 5, répondant à la formule générale (X) suivante :

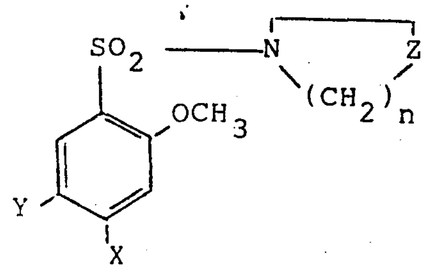

(X)

dans laquelle :

- n représente 2 ou 3,
- X représente H, $NO_2$, $OCH_3$,
- Y représente H, Cl, Br, $OCH_3$, $SO_2CH_3$, $SO_2iC_3H_7$, $CF_3$,
- Z a la signification indiquée dans les revendications 1 à 5.

7. Composés selon l'une quelconque des revendications 1 à 5, répondant à la formule générale (XI) suivante :

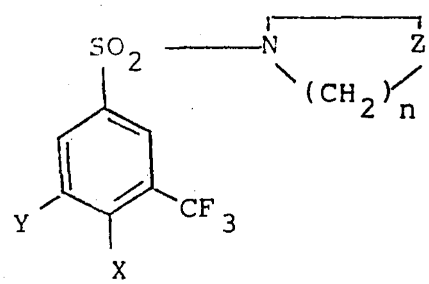

(XI)

dans laquelle :
- n vaut 2 ou 3,
- X représente H, Cl,
- Y représente H, $CF_3$,
- Z a la signification indiquée dans les revendications 1 à 5.

8. Composés de formules suivantes :

Composés n°

1 242

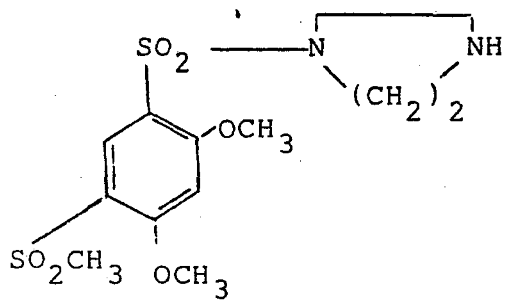

Composé n°

1 265

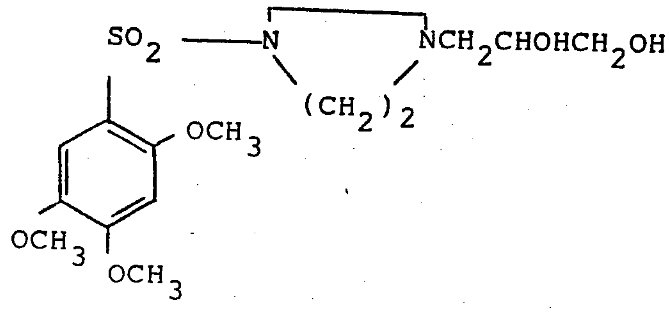

1 270

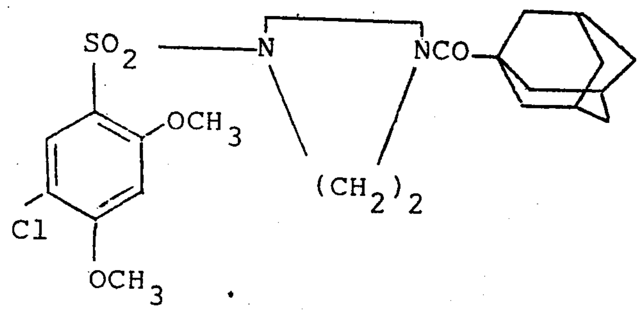

1 301

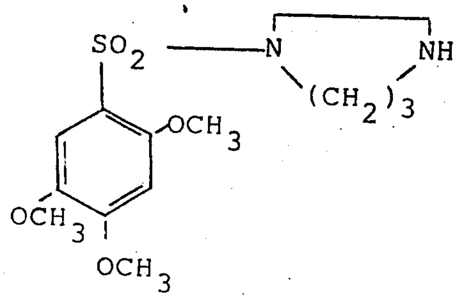

1 304

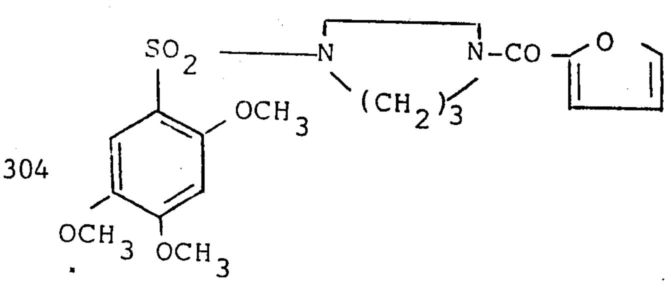

Composé n°

1 306

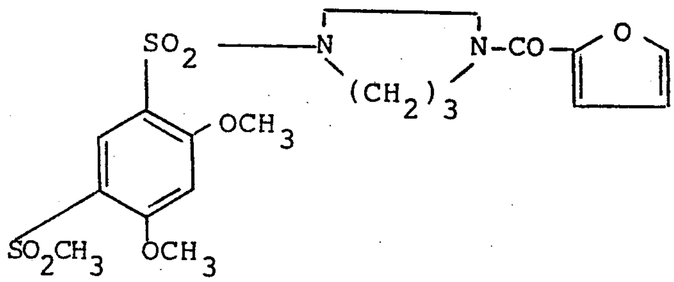

1 334

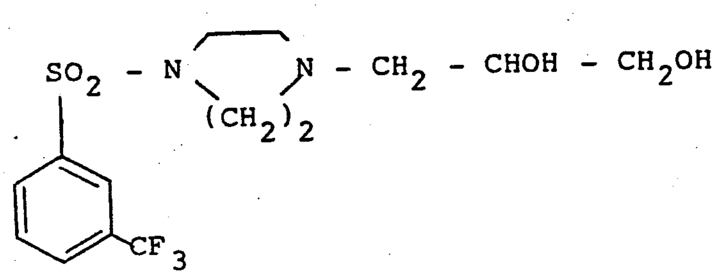

1 400

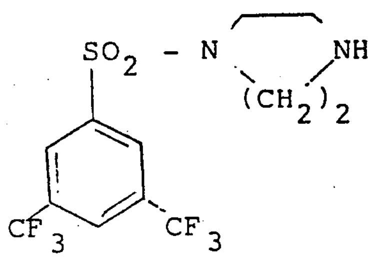

1 399

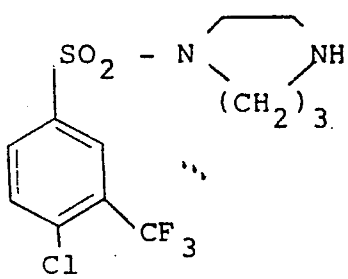

Composé n°

1 389

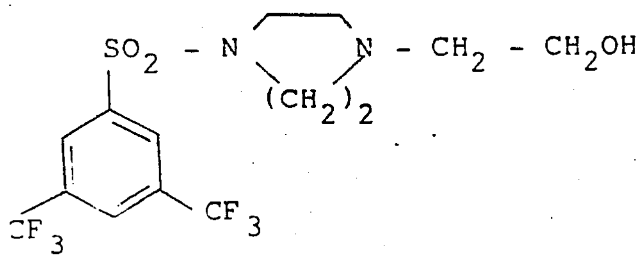

1 397

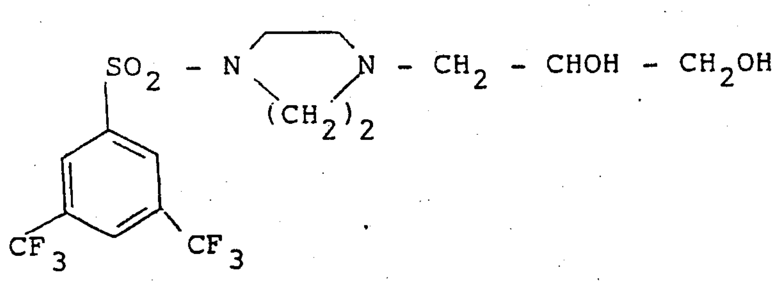

1 390

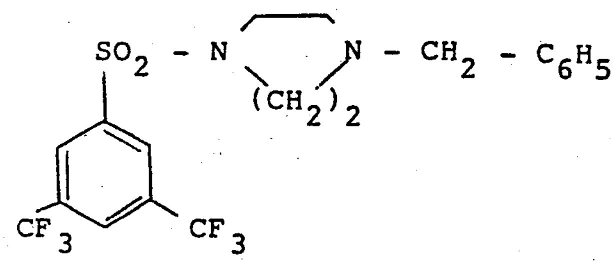

1 336

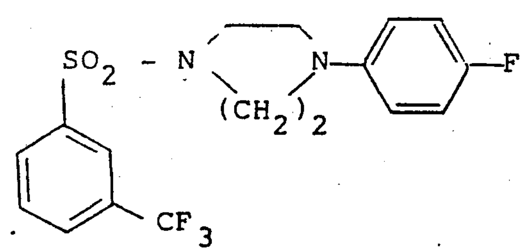

Composé n°

1 392

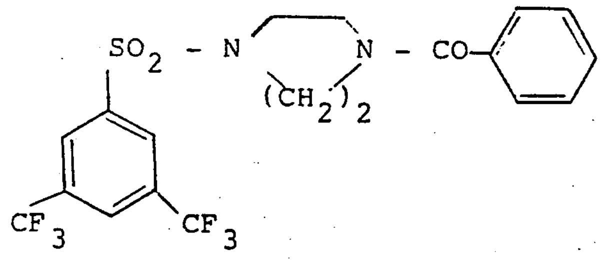

1 393

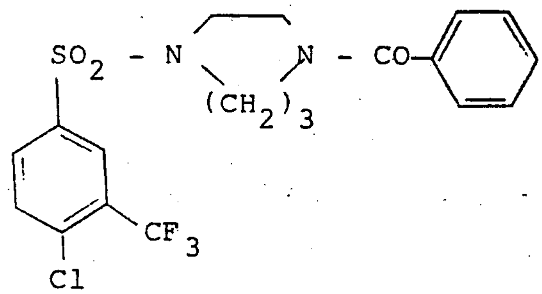

1 402

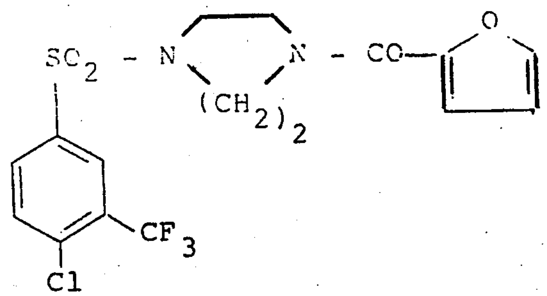

Composé n°

1 396

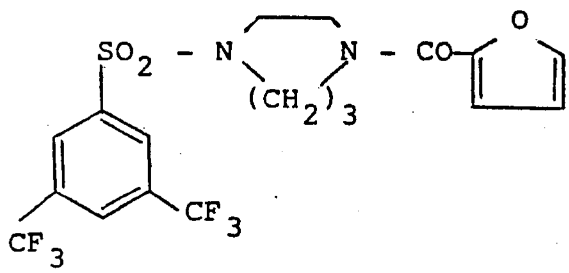

1 404

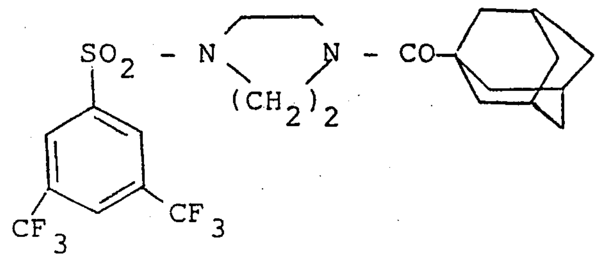

1 407

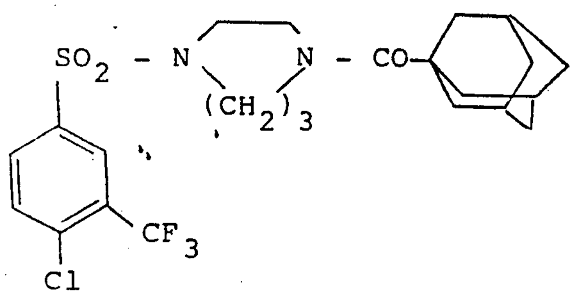

Composés
n°

1 426

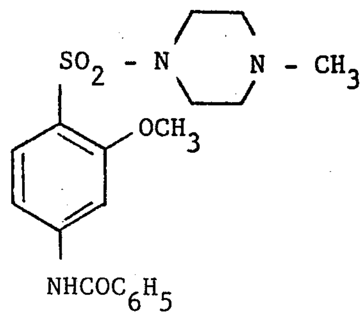

1 505

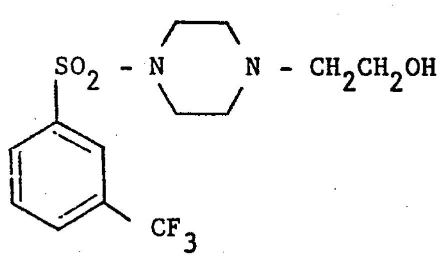

1 260

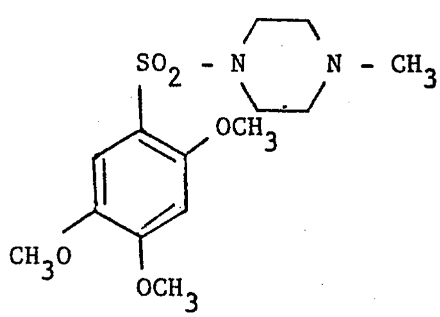

1 299 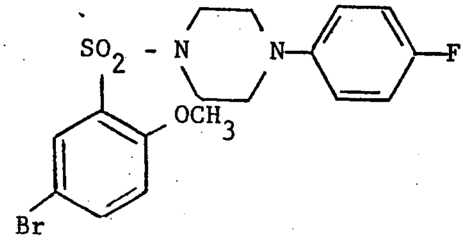

1 403 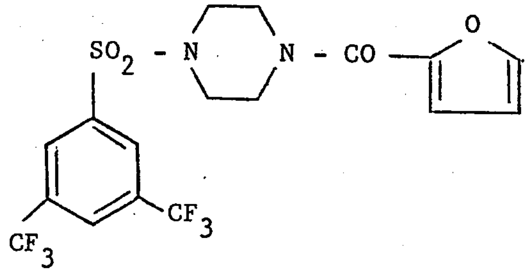

1 406 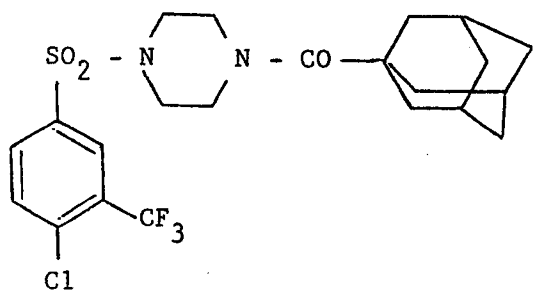

1 408 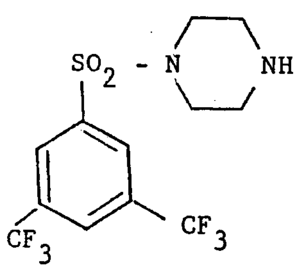

9. Procédé de préparation des composés selon l'une quelconque des revendications 1 à 8, caractérisé en ce que l'on fait réagir le composé de formule :

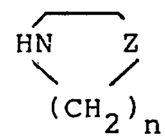

dans laquelle :

- n et Z ont les mêmes significations qu'à la revendication 1 ;

sur le sulfochlorure de formule :

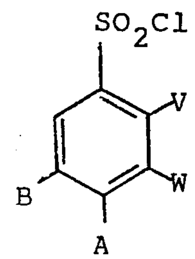

dans laquelle :

- V et W ont les mêmes significations qu'à la revendication 1 ;
- A a la même signification que X selon la revendication 1 à l'exception de $NH_2$ et de $NHCOR_2$ ;
- B a la même signification que Y selon la revendication 1 à l'exception de $NH_2$ et de $NHCOR_2$.

10. Procédé selon la revendication 9, de préparation de composés de formule :

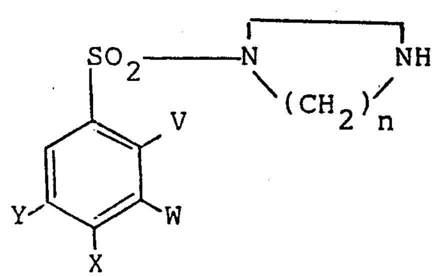

dans laquelle n, V, W, X et Y ont les significations indiquées à la revendication 1, caractérisé en ce que l'on

fait réagir le sulfochlorure de formule :

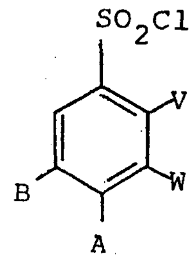

dans laquelle n, V, W ont les mêmes significations qu'à la revendication 1 et A et B ont les mêmes significations indiquées à la revendication 9 ;

- soit sur le composé de formule :

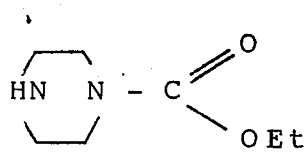

suivi d'une hydrolyse alcaline du composé obtenu par exemple par action d'une base forte, telle que KOH ;

- soit sur le composé de formule :

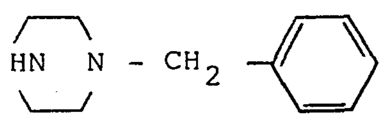

suivi d'une débenzylation par exemple par hydrogénolyse en présence de palladium et de carbone.

11. Procédé selon la revendication 9 d'obtention de composés de formule :

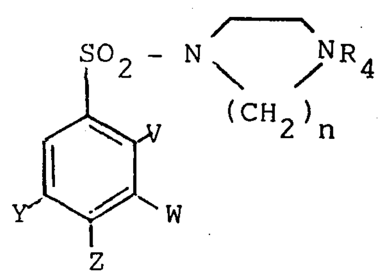

dans laquelle V, W, X, Y, n ont les significations indiquées à la revendication 1 et $R_4$ représente :

- un radical aralcoyle dans lequel la chaîne aliphatique présente de 1 à 4 atomes de carbone et peut comporter des groupes C = O et C-OH et dans lequel le groupe aryle est non substitué ou substitué par un halogène, par le groupe $CF_3$, par un radical $OR_1$, dans lequel $R_1$ est un radical alcoyle ayant de 1 à 4 atomes de carbone ;

- un radical aroyle ou ses isostères non substitués ou substitués par un halogène par le groupe $CF_3$, par un radical $OR_1$, dans lequel $R_1$ est un groupe alcoyle ayant de 1 à 4 atomes de carbone ;

- un radical hydroxyalcoyle ayant de 1 à 3 atomes de carbone ;

par action de :

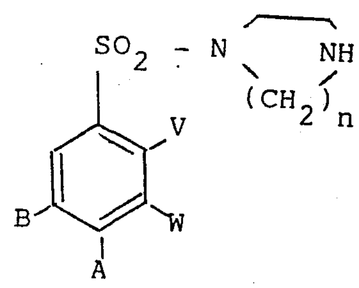

dans lequel n, V, W ont les significations indiquées à la revendication 1 et A et B ont les significations indiquées à la revendication 9 ;

respectivement sur :

- le chlorure d'aralcoyle correspondant,
- le chlorure d'aroyle correspondant,
- ou sur l'époxy correspondant.

12. Procédé selon la revendication 9, caractérisé en ce que l'on transforme le composé de formule :

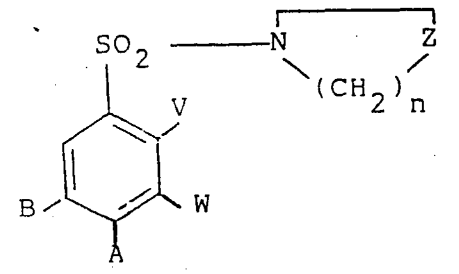

dans laquelle n, V, W ont les significations indiquées à la revendication 1, l'un des deux substituants A ou B représente $NO_2$ et l'autre a la signification indiquée à la revendication 9, en composé de formule :

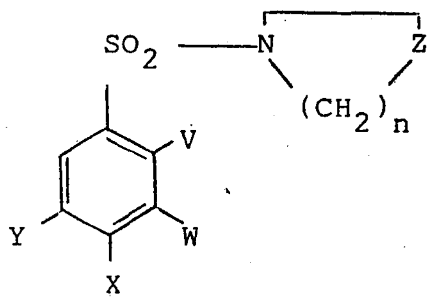

dans laquelle n, V, W ont les significations indiquées à la revendication 1, l'un des deux substituants X ou Y représente $NH_2$ ou $NHCOR_2$ et l'autre a la signification indiquée à la revendication 1, par réduction catalytique ou chimique pour transformer $NO_2$ en $NH_2$, puis si besoin est par action de $R_2COCl$ ou :

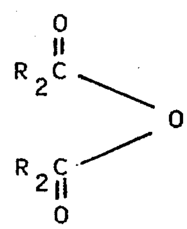

pour transformer $NH_2$ en $NH\underset{\underset{O}{\|}}{C}R_2$.

13. Procédé selon l'une quelconque des revendications 9 à 12, caractérisé en ce que le composé de formule :

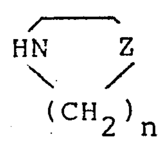

est choisi parmi :

- la morpholine,
- la pipéridine,
- la pipérazine,
- l'homopipérazine,
- la N(hydroxy-2)éthyl pipérazine,
- la N-benzylpipérazine,
- la (fluoro-4 phényl)-1 pipérazine,
- la (dihydroxy-2,3 propyl)-1 pipérazine,
- la N-méthyl pipérazine,
- la N-carbéthoxypipérazine,
- l'(hydroxy-3-propyl)-1 pipérazine.

14. Procédé selon l'une quelconque des revendications 9 à 12, caractérisé en ce que le chlorure de formule :

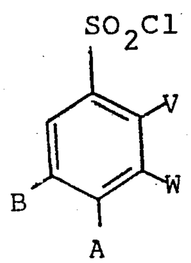

est choisi parmi le :

- chlorure de (ditrifluorométhyl-3,5) benzènesulfonyle ;
- chlorure de bromo-5 diméthoxy-2,4 benzènesulfonyle ;
- chlorure de chloro-5 méthoxy-2 benzènesulfonyle ;
- chlorure de chloro-5 diméthoxy-2,4 benzènesulfonyle ;
- chlorure de diméthoxy-2,4 isopropyl-sulfonyl-5 benzènesulfonyle ;
- chlorure de diméthoxy-2,4 méthyl-sulfonyl-5 benzènesulfonyle.

15. Composition pharmaceutique, caractérisée en ce qu'elle contient, comme principe actif, l'un au moins des composés selon l'une quelconque des revendications 1 à 8 en association avec un véhicule pharmaceutiquement

acceptable.

16. Composition pharmaceutique, caractérisée en ce qu'elle contient en association avec un véhicule pharmaceutiquement acceptable un principe actif constitué par l'un au moins des composés de formule (I) :

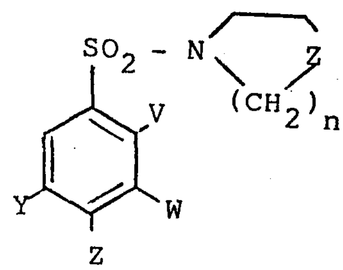

(I)

dans laquelle :

- V représente l'hydrogène, un radical $OR_1$ dans lequel $R_1$ représente un groupe alcoyle de 1 à 4 atomes de carbone ;

- W représente l'hydrogène, le groupe $CF_3$ ;

- X représente l'hydrogène, un halogène, le groupe $NO_2$, $NH_2$, un radical $NH-CO-R_2$ dans lequel $R_2$ représente un radical alcoyle de 1 à 4 atomes de carbone ou aryle, un radical $OR_1$ dans lequel $R_1$ représente un radical alcoyle de 1 à 4 atomes de carbone ;

- Y représente l'hydrogène, un halogène, le groupe $NO_2$, $NH_2$, $CF_3$, un radical $NH-CO-R_2$ dans lequel $R_2$ représente un radical alcoyle de 1 à 4 atomes de carbone ou aryle, un radical $OR_1$ dans lequel $R_1$ représente un radical alcoyle de 1 à 4 atomes de carbone, un radical $SO_2-R_3$ dans lequel $R_3$ représente un radical alcoyle de 1 à 4 atomes de carbone,

- n vaut 2 ou 3 ;

- Z représente un atome d'oxygène, le groupe $CH_2$ ou $NR_4$ dans lequel :

. $R_4$ représente :

- un hydrogène,

- un radical alcoyle inférieur de 1 à 6 atomes de carbone,

- un radical hydroxyalcoyle de 1 à 3 atomes de carbone,

- un radical cycloalcanoyle de 3 à 10 atomes de carbone,

- un radical aryle non substitué ou substitué par un halogène, par le groupe $CF_3$, par un radical $OR_1$ dans lequel $R_1$ représente un radical alcoyle ayant de 1 à 4 atomes de carbone,

- un radical aralcoyle dans lequel la chaîne aliphatique présente de 1 à 4 atomes de carbone et peut comporter des groupes C = O et C-OH dans lequel le groupe aryle est non substitué ou substitué par un halogène, par le groupe $CF_3$, par un radical $OR_1$, dans lequel $R_1$ est un radical alcoyle ayant de 1 à 4 atomes de carbone,

- un radical aroyle ou ses isostères non substitués ou substitués par un halogène, par le groupe $CF_3$, par un radical $OR_1$ dans lequel $R_1$ est un groupe alcoyle de 1 à 4 atomes de carbone ;

sous réserve que lorsque n = 2 :

- soit 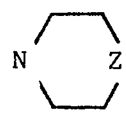 représente un groupe 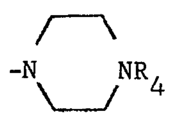, $R_4$ ayant la signification ci-dessus indiquée, et W ou Y représente un groupe $CF_3$ ;

- soit V représente un radical $OR_1$, $R_1$ étant un groupe alcoyle de 1 à 4 atomes de carbone, l'un au moins des éléments W, X ou Y étant différent de l'hydrogène.

17. Composition pharmaceutique, caractérisée en ce qu'elle contient, en association avec un véhicule pharmaceutiquement acceptable, un principe actif constitué par l'un au moins des composés de formule :

Composés n°

1 211

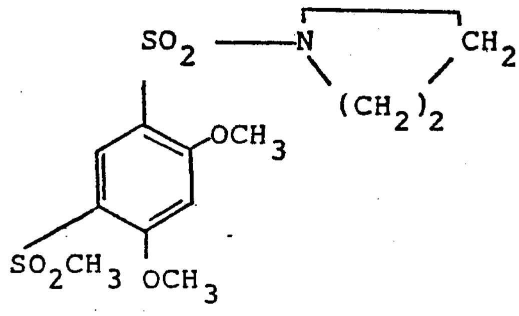

1 224

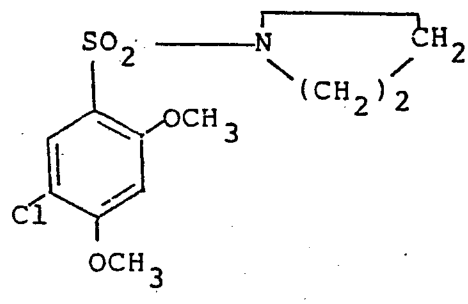

1 242

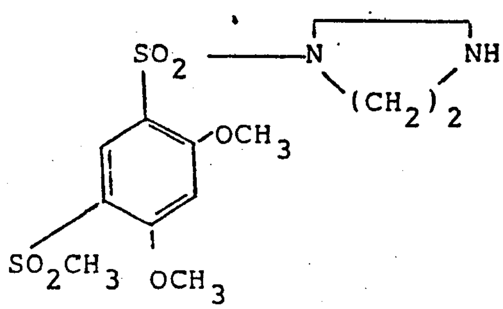

1 247

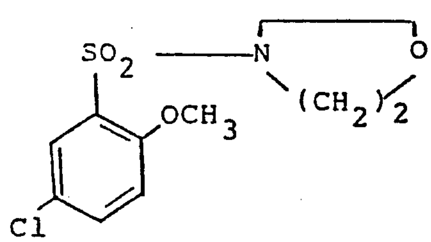

Composé n°

1 265

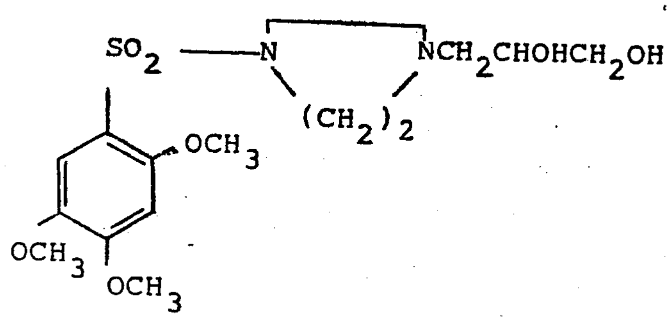

1 270

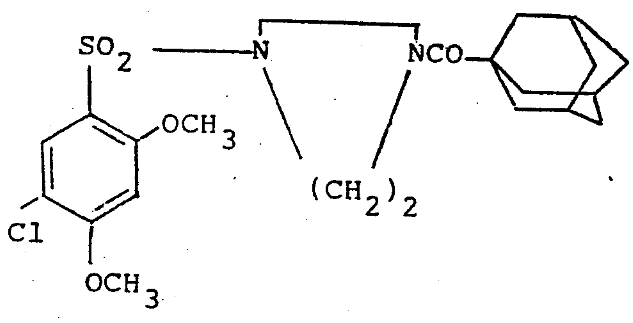

1 301

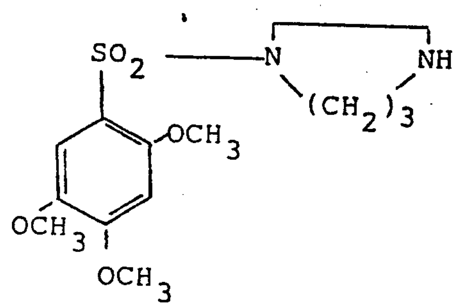

1 304

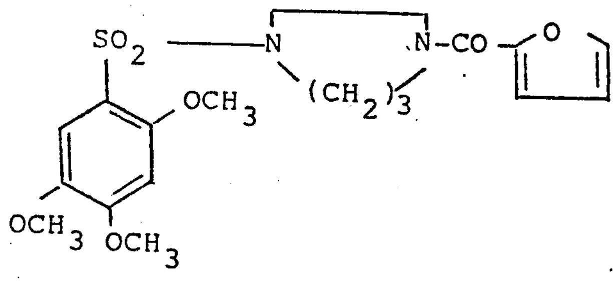

Composé n°

1 306

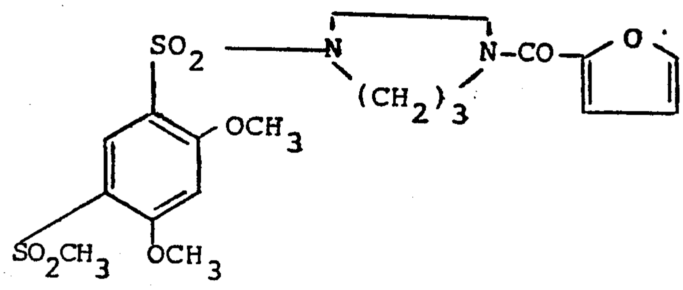

1 334

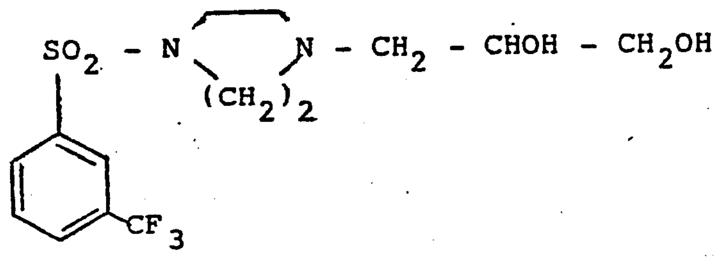

1 400

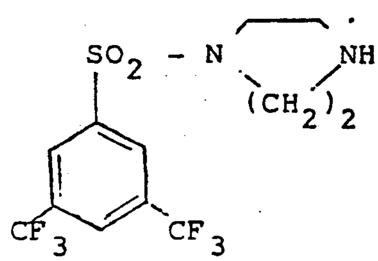

1 399

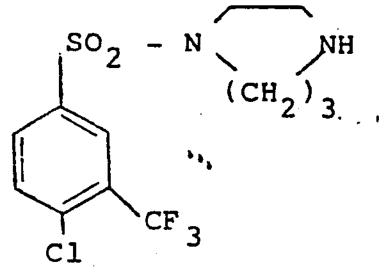

Composé n°

1 389

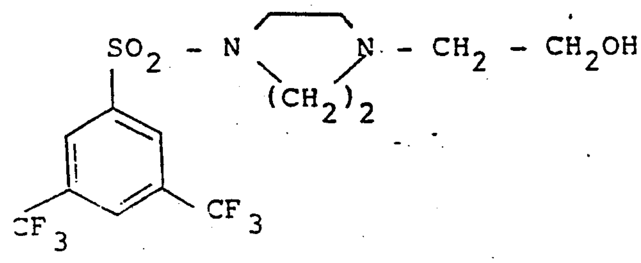

1 397

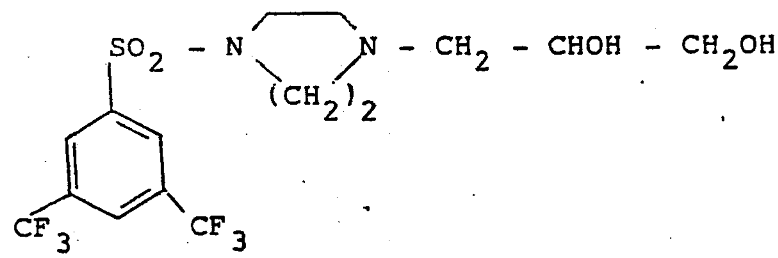

1 390

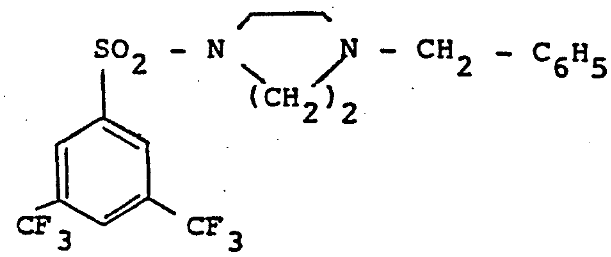

1 336

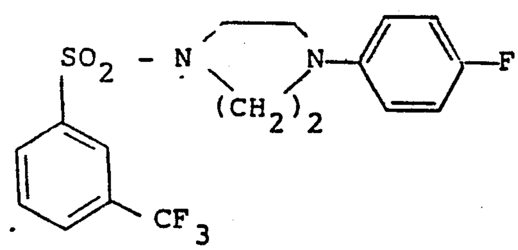

Composé n°

1 392

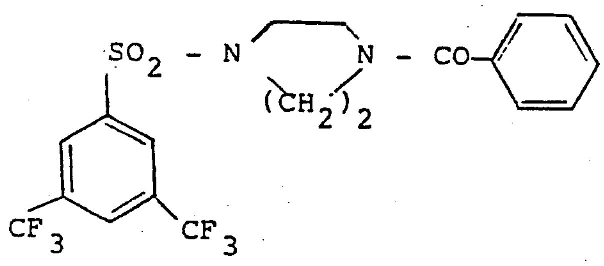

1 393

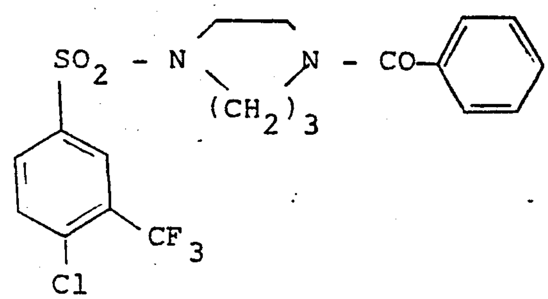

1 402

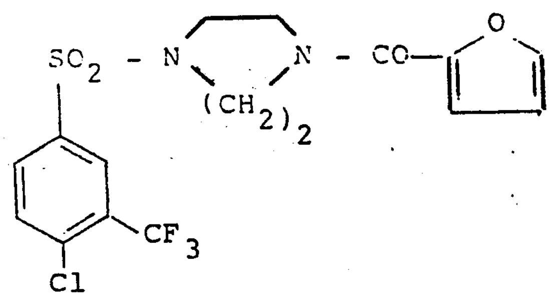

Composé n°

1 396

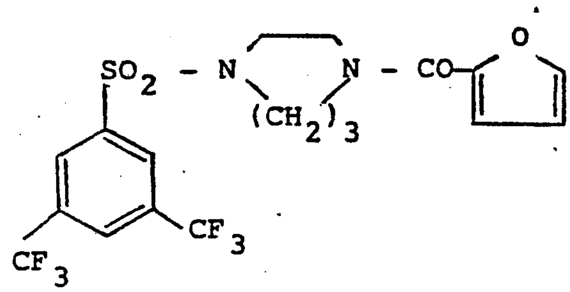

1 404

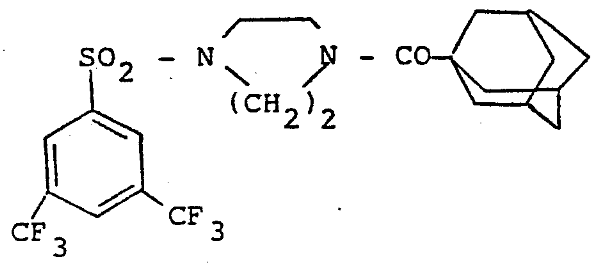

1 407

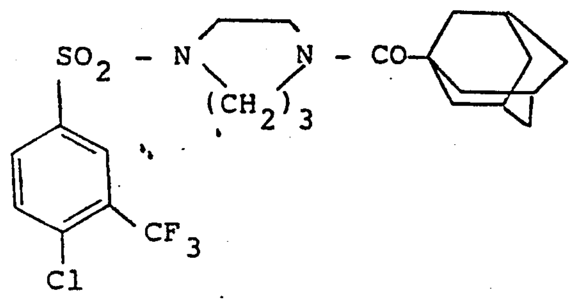

Composés n°

1 426

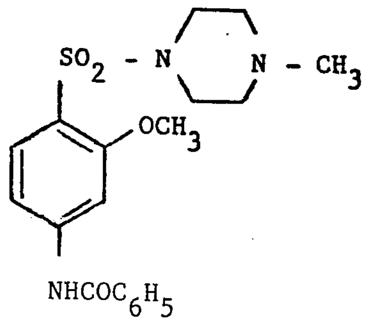

1 406

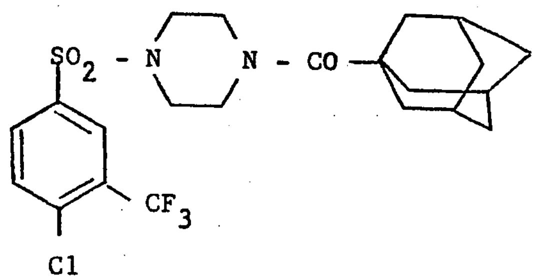

1 505

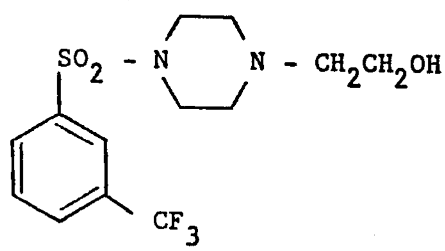

1 260

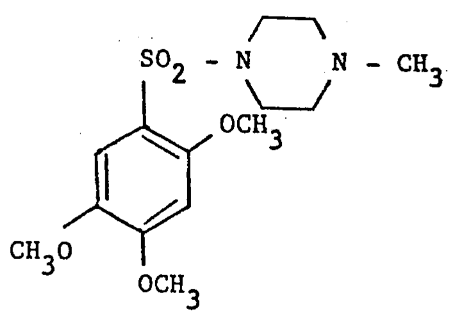

0138720

1 299

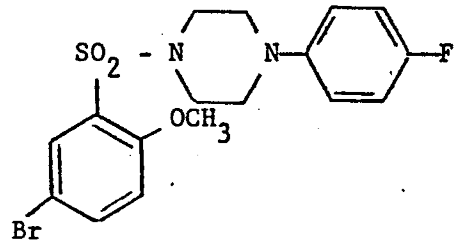

1 403

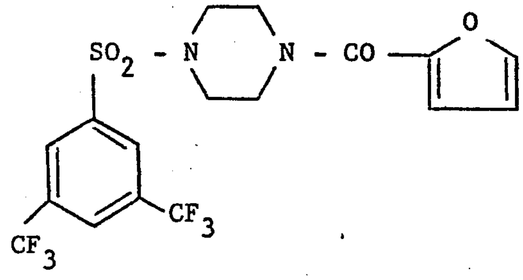

1 408

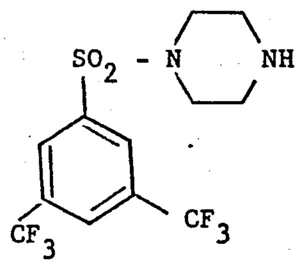

18. Composition pharmaceutique selon les revendications 15 à 17, caractérisée en ce qu'elle est dosée à des concentrations en principe actif susceptibles d'être administrées à raison d'environ 1 à environ 1 000 mg, notamment d'environ 5 à environ 250 mg du susdit principe actif par jour.

19. Composition pharmaceutique selon l'une quelconque des revendications 15 à 18, caractérisée en ce que le conditionnement unitaire se présente sous une forme administrable par voie orale, notamment comprimé ou gélule, contenant 5 à 10 mg de principe actif en association avec un véhicule pharmaceutiquement acceptable.